# EUROPÄISCHE PATENTANMELDUNG

(11) **EP 1 808 181 A1**
(43) Veröffentlichungstag der Anmeldung: **18.07.2007**
(21) Anmeldenummer: 06100438.8
(22) Anmeldetag: 17.01.2006
(51) Int. Cl.: A61K 47/48

(54) **Polysaccharid-Cyclodextrin-Konjugate**

(71) Anmelder: B. BRAUN MELSUNGEN AG, 34212 Melsungen (DE)
(72) Erfinder: Fisch, Andreas, 1005, Lausanne (CH); Schmitt, Jürgen, 35274 Kirchhain (DE)
(74) Vertreter: Weber, Thomas

(57) **Zusammenfassung**

Die vorliegende Erfindung betrifft wasserlösliche Polysaccharid-Cyclodextrin-Addukte umfassend ein Polysacharid (PS) und wenigstens ein Cyclodextrin (CD), die über wenigstens eine Linker-Gruppe kovalent miteinander verknüpft sind. Die Erfindung betrifft zudem pharmazeutische Zusammensetzungen umfassend die erfindungsgemäßen Addukte und die Verwendung der Addukte zur Solubilisierung von pharmazeutischen Wirkstoffen. Demgemäß betrifft die Erfindung ferner Zusammensetzungen umfassend die erfindungsgemäßen Addukte und wenigstens einen pharmazeutischen Wirkstoff.

## Beschreibung

Die vorliegende Erfindung betrifft wasserlösliche Polysaccharid-Cyclodextrin-Addukte umfassend ein Polysaccharid (PS) und wenigstens ein Cyclodextrin (CD), die über wenigstens eine Linker-Gruppe kovalent miteinander verknüpft sind. Die Erfindung betrifft zudem pharmazeutische Zusammensetzungen umfassend die erfindungsgemäßen Addukte und die Verwendung der Addukte zur Solubilisierung von pharmazeutischen Wirkstoffen. Demgemäß betrifft die Erfindung ferner Zusammensetzungen umfassend die erfindungsgemäßen Addukte und wenigstens einen pharmazeutischen Wirkstoff.

### Technischer Hintergrund der Erfindung

Cyclodextrine (CD) und ihre Derivate sind cyclische Oligosaccharide, die komplexierende Eigenschaften für eine Vielzahl von Arzneistoffen, insbesondere niedermolekulare klassische Wirkstoffe, aber auch zum Teil höhermolekulare Wirkstoffe wie Proteine/Peptide, aufweisen.

Albers et al (Critical Reviews in Therapeutic Drug Carrier Systems, 12(4): 311-337) und Stella et al (Pharmaceutical Research Vol. 14, No. 5, 1997) beschreiben den Einsatz von CD zur Solubilisierung von Arzneistoffen. Dabei wird durch deren Komplexierung mit CD die Wasserlöslichkeit der Pharmaka und somit ihre Aufnahme in den Organismus gesteigert. Aufgrund der relativ geringen Affinität der CD zu den meisten Wirkstoffen, erfolgt deren Freisetzung in der Regel bereits unmittelbar nach der Injektion aufgrund von Verdünnungseffekten im Plasma, oder auch durch kompetitive Verdrängung durch endogene Substanzen (plasmatisches Cholesterol etc.). Die infundierten CD werden im Organismus in der Regel nicht metabolisiert und rasch (innerhalb weniger Stunden) hauptsächlich renal eliminiert.

Damit wären CD als Hilfsstoffe zur Solubilisierung schwer löslicher Arzneistoffe in topischen, enteralen und parenteralen Arzneiformen prinzipiell geeignet, wenn nicht ihre Toxizität (Nierenschädigung durch Auskristallisation; Cytotoxizität durch Komplexierung von Membranlipiden und Cholesterin) deren pharmazeutische Anwendung, besonders in Parenteralias, deutlich einschränken würde. Infolgedessen muss die zum Einsatz kommende Dosierung der CD stark reduziert werden, wodurch allerdings die Verabreichung größerer Wirkstoffmengen stark erschwert wird.

Der Erfindung liegt daher die Aufgabe zugrunde nichttoxische Verbindungen bereitzustellen, die eine Solubilisierung von in physiologischen Medien, insbesondere in Wasser unlöslichen oder schlecht löslichen Wirkstoffen ermöglichen.

Überraschenderweise wurde im Rahmen der vorliegenden Untersuchungen festgestellt, dass durch die kovalente Anbindung handelsüblicher Cyclodextrine an physiologisch unbedenkliche Polymere, insbesondere an Polysaccharide, eine Detoxifizierung der Cyclodextrine erfolgt, ohne deren Solubilisierungsvermögen zu beeinträchtigen. Die Detoxifizierung der Cyclodextrine, i.e. die Verminderung der Zytotoxizität, konnte insbesondere anhand verminderter Hämolyseraten gezeigt werden.

### Beschreibung der Figuren

In Figur 1 werden Syntheserouten zur Herstellung geeigneter funktionalisierter CD exemplarisch und nicht abschließend dargestellt.

In Figur 2 werden Syntheserouten zur Herstellung geeigneter Stärken exemplarisch und nicht abschließend dargestellt.

In Figur 3 werden weitere geeignete Linker und deren Kombinationen mit Cyclodextrinen nicht abschließend beschrieben.

### Beschreibung der Erfindung

Die vorliegende Erfindung betrifft eine wasserlösliche Verbindung umfassend ein Polysaccharid (PS) und wenigstens ein Cyclodextrin (CD), die über wenigstens eine Linker-Gruppe kovalent miteinander verknüpft sind. Die erfindungsgemäßen Verbindungen werden infolge auch als Addukte bezeichnet.

Ein zweiter Aspekt der vorliegenden Erfindung betrifft Verfahren zur Herstellung der obigen Addukte durch kovalente Anknüpfung eines Polysaccharids an ein Cyclodextrin über eine Linker-Gruppe.

Ein alternativer Aspekt der vorliegenden Erfindung betrifft ein Verfahren zur Herstellung der obigen Addukte durch Reaktion eines Polysaccharids und eines Cyclodextrins mit einem bifunktionellen Linkermolekül.

Ein weiterer Aspekt der vorliegenden Erfindung betrifft eine pharmazeutische Zusammensetzung umfassend die erfindungsgemäßen Addukte.

Ein weiterer Aspekt der Erfindung betrifft die Verwendung der erfindungsgemäßen Addukte zur Solubilisierung von in Wasser schlecht löslichen bzw. unlöslichen Wirkstoffen, insbesondere von pharmazeutischen Wirkstoffen.

Bei den erfindungsgemäßen Addukten handelt es sich um wasserlösliche Verbindungen umfassend einen Kolloidbestandteil, vorzugsweise einen Polysaccharidbestandteil (PS) und einen Cyclodextrinbestandteil (CD), die über wenigstens eine Linker-Gruppe (L) umfassend -L₁{-R-L₂}ₘ-, wobei L₁, L₂, m und R nachfolgend näher beschrieben werden, kovalent miteinander verknüpft sind. Dabei kann ein Polysaccharid-Molekül mit einer Vielzahl von Cyclodextrin-Molekülen verknüpft sein. Um die erforderliche Wasserlöslichkeit zu erzielen ist es vorteilhaft, eine Bildung von Aggregaten umfassend mehrere Polysaccharidbestandteile (PS) zu vermeiden.

Wasserlöslich bedeutet im Zusammenhang mit der vorliegenden Erfindung, dass bei Raumtemperatur (20°C) wenigstens 0,1 g bis wenigstens 0,2 g, vorzugsweise wenigstens 0,3 g bis wenigstens 0,4 g, besonders bevorzugt wenigstens 0,5 bis wenigstens 0,8 g der erfindungsgemäßen Addukte in 1 ml Wasser gelöst werden können.

Die erfindungsgemäßen Addukte weisen (ohne den komplexierten Wirkstoff) ein mittleres Molekulargewicht M_{w} zwischen 10 und 500 kDa, vorzugsweise M_{w} zwischen 20 und 100 kDa, besonders bevorzugt zwischen 40 und 60 kDa auf.

Die erfindungsgemäßen Addukte können durch die allgemeine Formel I beschrieben werden:

[(CD)-L₁{-R-L₂}ₘ]ₙ-(PS) (I)

In Formel I bedeutet:
- (CD) ein Cyclodextrin;
- (PS) eine Polysaccharid;
- L₁ und L₂ können gleich oder unterschiedlich sein und jeweils eine Linker-Gruppe darstellen;
- R ist eine Brückengruppe von geeigneter Länge, welche im einfachsten Fall eine Einfachbindung sein kann. Ferner kann R ausgewählt sein aus der Gruppe bestehend aus einer linearen oder verzweigten, gesättigten oder ungesättigten, aliphatischen oder alicyclischen Kohlenwasserstoffgruppe mit 1 bis 22, vorzugsweise 2 bis 15, besonders bevorzugt 3 bis 8 Kohlenstoffatomen, die eine oder mehrere Doppelbindungen und zusätzliche Heteroatome, die ausgewählt sind aus der Gruppe bestehend aus O, S, N, Se, Si und P, sowie funktionellen Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatome enthalten können; einer aromatischen Gruppe enthaltend 5 bis 14, vorzugsweise 6 bis 12, besonders bevorzugt 6 bis 10 Kohlenstoffatome; einer gesättigten oder ungesättigten heterocyclischen Gruppe enthaltend 3 bis 10, vorzugsweise 4 bis 8, besonders bevorzugt 5 bis 6 Kohlenstoffatome und wenigstens ein Heteroatom ausgewählt aus O, S, N, Se, Si und P; einer Aryl-C₁₋₆-alkylen-Gruppe mit 5 bis 12, besonders bevorzugt 6 bis 10 Kohlenstoffatomen im Arylrest, die substituiert sein können mit wenigstens einer C₁₋₈-Alkylen-, vorzugsweise C₂₋₆-Alkylen-, besonders bevorzugt C₃₋₄-Alkylen-Gruppe, wobei sowohl Arylrest als auch Alkylen-Gruppe eine oder mehrere Doppelbindungen, zusätzliche Heteroatome, die ausgewählt sind aus der Gruppe bestehend aus O, S, N, Se, Si und P, sowie funktionelle Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatomen aufweisen können;
- m ist gleich 0 oder 1 und
- n ist eine ganze Zahl zwischen 1 und 10000, vorzugsweise zwischen 100 und 5000, besonders bevorzugt zwischen 1000 und 3000.

### Cyclodextrine (CD)

Im Zusammenhang mit der vorliegenden Erfindung bestehen keine Einschränkungen hinsichtlich der zu verwendenden Cyclodextrine (CD). Vorzugsweise werden CD und funktionalisierte CD eingesetzt, die ausgewählt sind aus Verbindungen, die wenigstens ein Strukturelement gemäß der folgenden allgemeinen Formel II umfassen:

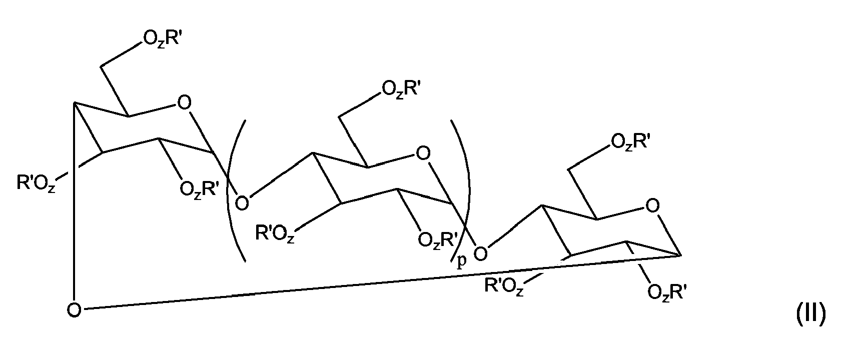

In Formel II ist p eine ganze Zahl, die ausgewählt ist aus 4, 5 oder 6, wobei Cyclodextrine mit p = 4 und 5 im Hinblick auf ihre Komplexierungseigenschaften für Wirkstoffe bevorzugt sind.

Die Reste R' sind unabhängig voneinander ausgewählt aus der Gruppe bestehend aus
- Wasserstoff;
- verzweigten und linearen Alkyl-Gruppen mit 1 bis 10, vorzugsweise 2 bis 6, besonders bevorzugt 3 bis 5 Kohlenstoffatomen;
- Carboxy(alkyl)- oder Carboxyl-Gruppen der Formel -(CH₂)_{q}-COOH, wobei q eine ganze Zahl zwischen 0 und 6, vorzugsweise zwischen 1 und 5, besonders bevorzugt 1, 2 oder 3 ist;
- Sulfo(alkyl)ether-Gruppen der Formel -(CH₂)ᵣSO₃M, wobei r eine ganze Zahl zwischen 0 und 6, vorzugsweise zwischen 1 und 5, besonders bevorzugt 1, 2 oder 3 ist und M ausgewählt ist aus Na, K und Li;
- Hydroxyalkyl-Gruppen der allgemeinen Formel -(CH₂)ₛ(CHR")-OH, wobei s eine ganze Zahl zwischen 1 und 6, vorzugsweise zwischen 2 und 5, besonders bevorzugt 1, 2 oder 3 ist und R" ausgewählt ist aus Wasserstoff und C₁₋₅-Alkyl-Gruppen, vorzugsweise ausgewählt aus Wasserstoff und einer Methyl-Gruppe ist;
- Amino(alkyl)-Gruppen der Formel -(CH₂)ₜ-NH₂, wobei t eine ganze Zahl zwischen 0 und 6, vorzugsweise zwischen 1 und 5, besonders bevorzugt 1, 2 oder 3 ist;
- Isocyanato(alkyl)-Gruppen der Formel -(CH₂)ᵤ-NCO, wobei u eine ganze Zahl zwischen 0 und 6, vorzugsweise zwischen 2 und 5, besonders bevorzugt 1, 2 oder 3 ist;
- Mercapto(alkyl)-Gruppen der Formel -(CH₂)ᵥ-SH, wobei v eine ganze Zahl zwischen 0 und 6, vorzugsweise zwischen 2 und 5, besonders bevorzugt 1, 2 oder 3 ist; und
- Halogenatomen ausgewählt aus Cl, Br und I.

Unabhängig von den übrigen Resten ist z = 0 oder 1, vorzugsweise 1. Für den Fall, dass q, r, s, t, u oder v = 0 ist, ist z vorzugsweise ebenfalls gleich 0.

Der Fall, bei dem für alle Substituenten des Cyclodextrins z = 0 und R' = H ist, führt zu unfunktionalisierten Cyclodextrinen und soll im Zusammenhang mit der vorliegenden Erfindung ausgeschlossen werden.

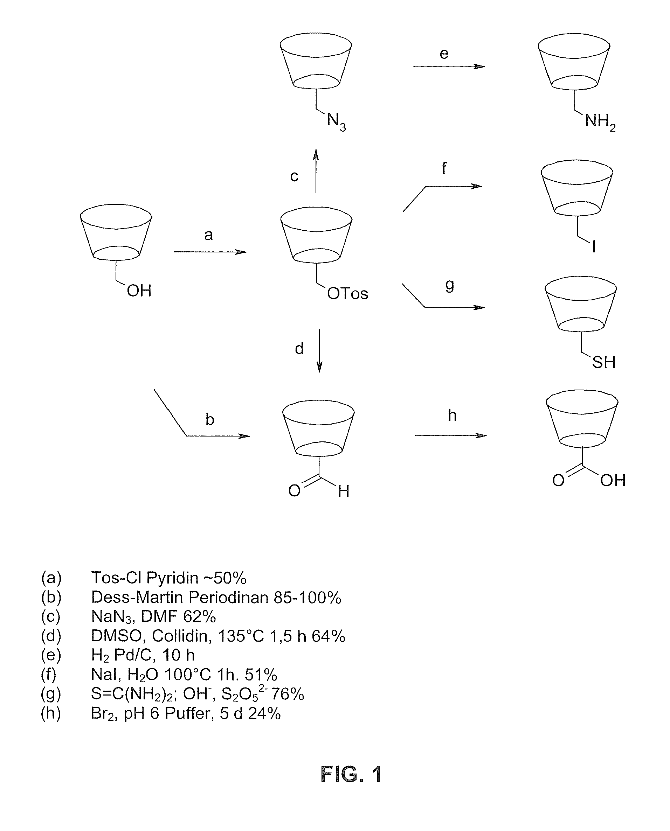

Syntheserouten zur Herstellung geeigneter funktionalisierter CD sind exemplarisch und nicht abschließend in Figur 1 dargestellt.

Die in Figur 1 dargestellten Syntheseschritte (a), (c), (e) und (f) werden in L.D. Melton and K.N. Slessor, Carbohydr. Res., 1971 (18) 29-33; der Syntheseschritt (b) in Cornwell M.J., Huff J.B., Bieniarz C. Tet. Lett. 1995 36 8371-8374; die Schritte (d) und (h) in Yoon J., Hong S., Martin K.A., Czarnik A.W. J. Org. Chem. 1995, 60, 2792-2795 und der Schritt (g) in Siegel B., J. Inorg. Nucl. chem. 1979 (41) 609-610 beschrieben.

Beispiele geeigneter Cyclodextrine sind α-Cyclodextrin (z = 1, p = 4, R' = H), β-Cyclodextrin (z = 1, p = 5, R' = H), γ-Cyclodextrin (z = 1, p = 6, R' = H), Carboxymethyl-β-cyclodextrin (z = 1, p = 5, R' = CH₂COOH), Carboxymethyl-ethyl-β-cyclodextrin (z = 1, p = 5, R' = CH₂COOH, C₂H₅ oder H), Diethyl-β-cyclodextrin (z = 1, p = 5, R' = C₂H₅ oder H), Dimethyl-β-cyclodextrin (z = 1, p = 5, R' = CH₃ oder H), Methyl-β-cyclodextrin (z = 1, p = 5, R' = CH₃ oder H), randomisiertes Methyl-β-cyclodextrin (z = 1, p = 5, R' = CH₃ oder H), Glucosyl-β-cyclodextrin (z = 1, p = 5, R' = Glucosyl oder H), Maltosyl-β-cyclodextrin (z = 1, p = 5, R' = Maltosyl oder H), Hydroxyethyl-β-cyclodextrin (z = 1, p = 5, R' = CH₂CH₂OH oder H), Hydroxypropyl-β-cyclodextrin (z = 1, p = 5, R' = CH₂(CHOH)CH₃ oder H) und Sulfobutylether-β-cyclodextrin (z = 1, p = 5, R' = (CH₂)₄SO₃Na oder H).

CD sind kommerziell, beispielsweise unter den Handelsnamen Cavamax® (Methyl-β-cyclodextrin), Cavasol® W7 MCT (Monochlortriazinyl-β-cyclodextrin) und Cavasol® (Hydroxypropyl-β-cyclodextrin) der Firma Wacker, AG, Deutschland oder unter der Bezeichnung Cleptose® (Methyl-β-cyclodextrin) von der Firma Roquette, Frankreich erhältlich.

Die Funktionalisierung von CD kann an den Hydroxy-Gruppen an C-6, C-2 und C-3 der Glucoseeinheiten der CD erfolgen. Gemäß der vorliegenden Erfindung können sowohl einfach als auch mehrfach funktionalisierte CD Einsatz finden. In den folgenden Reaktionsschemata werden CD mit Hilfe der nachfolgenden Formel IIa dargestellt

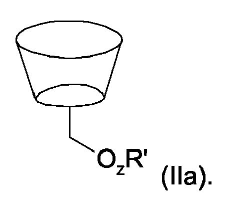

In Formel IIa wurde lediglich eine der Hydroxy-Gruppe an C-6 explizit dargestellt. Die übrigen Hydroxy-Gruppen an C-2 und C-3 sollen jedoch ausdrücklich umfasst sein. Gleichermaßen repräsentiert der Kegelstumpf der Formel IIa alle erfindungsgemäß einsetzbaren Ringgrößen (p= 4, 5 oder 6) der Cyclodextrine.

### Polysaccharide (PS)

Bei den zur Herstellung der erfindungsgemäßen Addukte verwendeten Polysacchariden (PS) handelt es sich um Verbindungen, die ausgewählt sind aus der Gruppe bestehend aus kationischen, anionischen und neutralen Stärken, und Stärkederivaten, die beispielsweise ausgewählt sind aus der Gruppe bestehend aus
(i) Carboxy(alkyl)- oder Carboxylstärken, umfassend wenigstens eine Carboxy(alkyl)- oder Carboxyl-Gruppe der Formel -(CH₂)_{q'}-COOR¹, wobei q' eine ganze Zahl zwischen 0 und 6, vorzugsweise zwischen 1 und 5, besonders bevorzugt 1, 2 oder 3 ist und R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist. Besonders bevorzugt sind Carboxymethylstärke (CMS), Carboxyethylstärke und Carboxypropylstärke;
(ii) Hydroxyalkyl-/ Mercaptoalkylstärken, umfassend wenigstens eine Hydroxyalkyl-/ Mercaptoalkyl-Gruppe der allgemeinen Formel -(CH₂)_{r'}(CHR"')-OH/-(CH₂)ᵣ,(CHR"')-SH, wobei r' eine ganze Zahl zwischen 1 und 6, vorzugsweise zwischen 2 und 5, besonders bevorzugt zwischen 3 und 4 ist; und R ausgewählt ist aus Wasserstoff und C₁₋₅-Alkyl-Gruppen, vorzugsweise Wasserstoff und Methyl-Gruppen, besonders bevorzugt ist Hydroxyethylstärke (HES);
(iii) Amino(alkyl)stärken und Aminoalkoxy(alkyl)stärken, umfassend wenigstens eine Aminoalkyl-Gruppe der Formel -(O)_{s'}(CH₂)ₓ₋NH₂, wobei s' eine ganze Zahl zwischen 0 und 6, vorzugsweise zwischen 1 und 5, besonders bevorzugt 1, 2 oder 3 und z = 0 oder 1 ist; und
(iv) Isocyanato(alkyl)stärken, umfassend wenigstens eine Isocyanato(alkyl)-Gruppen der Formel -(CH₂)_{t'}-NCO wobei t' eine ganze Zahl zwischen 0 und 6, vorzugsweise zwischen 1 und 5, besonders bevorzugt 1, 2 oder 3 ist;
(v) Tosyl-, Mesyl- und Triflat-Stärken umfassend p-Toluylsulfonyl-, Methylsulfonyl oder Trifluormethylsulfonyl-Gruppen;
sowie Amylose, Amylopektin, Aminostärken, Glycogen, Chitin, Inulin, Lektine, Glycoproteine, Dextrane und Alginate, die jeweils dahingehend modifiziert sein können, dass sie die zuvor unter (i) bis (v) bezeichneten funktionellen Gruppen aufweisen.

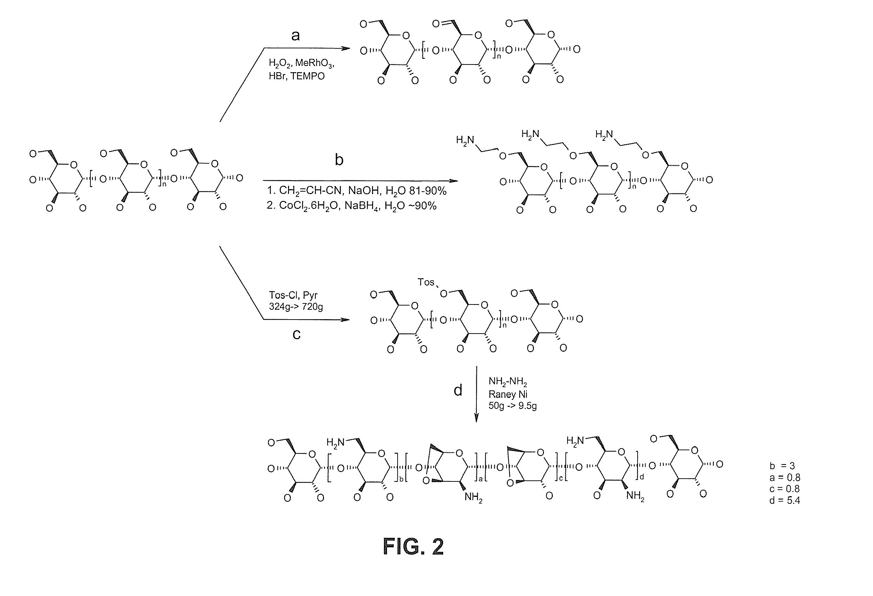

Syntheserouten zur Herstellung geeigneter Stärken sind exemplarisch und nicht abschließend in Figur 2 dargestellt.

Der in Figur 2 dargestellte Syntheseschritt (a) ist in Hermann W.A., Zoller J.P. Fischer R.W. J. Organometallic Chem. 1999 (579) 404-407, der Syntheseschritt (b) in Gonera A., Goclik V., Baum M., Mischnik P. Carbohydrate Res. 2002 (337) 2263-2272, die Schritte (c) und (d) sind in Wolfrom M.L., Taha M.I., Hopton D. J. Org. Chem. 1963 (28) 3553-3554 beschrieben.

Der Substitutionsgrad, Dₛ (Verhältnis der Anzahl modifizierter Anhydroglucoseeinheiten zur Anzahl der gesamten Anhydroglucoseeinheiten) der erfindungsgemäß verwendeten Stärken liegt im Bereich von 0,2 bis 0,8, vorzugsweise 0,3 bis 0,7, besonders bevorzugt 0,4 bis 0,5. Eine geeignete Methode zur Bestimmung des Dₛ wird von Sommermeyer et al in Krankenhauspharmazie, 8. Jahrg. Nr. 8, 1987, S. 274 beschrieben.

Im Zusammenhang mit der Erfindung bezeichnet der Begriff Polysaccharide makromolekulare Kohlenhydrate, welche aus 10 bis 50000, vorzugsweise 100 bis 10000, besonders bevorzugt 1000 bis 5000 glycosidisch miteinander verknüpften Monosaccharid Molekülen bestehen.

Idealerweise ist die Gesamtgröße der Polysaccharid-Cyclodextrin-Addukte so zu wählen, dass ihre Plasmaverweildauer so kurz wie möglich ist. Kurze Plasmaverweildauern werden im wesentlichen durch zwei mögliche Methoden erzielt:
(i) Das Molekulargewicht und der hydrodynamische Radius der Polysaccharid-Cyclodextrin-Addukte liegen unterhalb der Nierenschwelle (ca. 50000 Dalton);
(ii) Die Polysaccharid-Cyclodextrin-Addukte weisen ein Molekulargewicht und einen hydrodynamischen Radius oberhalb der Nierenschwelle auf, werden jedoch nach der Freisetzung des Wirkstoffs rasch in kleinere nierengängige Bruchteile gespalten.

Gemäß der Formel I ergeben sich zwei unterschiedliche Ausführungsformen in Abhängigkeit davon, ob m = 0 oder m = 1 ist.

### Linker-Gruppen (m=0)

Wenn m = 0 ist, wird der Linker L₁ durch die Reaktion einer freien funktionellen Gruppe des Cyclodextrins mit einer weiteren freien funktionellen Gruppe des Polysaccharids gebildet.

Der Begriff "freie funktionelle Gruppe" bedeutet im Zusammenhang mit der vorliegenden Erfindung, dass die Reaktivität der funktionellen Gruppe ausreicht, um eine kovalente Bindung auszubilden. Somit können auch "geblockte" funktionelle Gruppen freie funktionelle Gruppen im Sinne der Erfindung sein.

Die in dieser Alternative vorhandene Linker-Gruppe L₁ kann ausgewählt sein aus der Gruppe bestehend aus Ester- (-(C=O)O-) oder Thioester- (-(C=O)S-), Amid-(-(C=O)NH-), Urethan- (-NH-(C=O)O-), Carbamat (-NH-(C=O)O-), Thiocarbamat-(-NH-(C=O)S-), Carbonat- (-O-(C=O)-O-), Ether (-O-), Thioether (-S-), Aldimin (-HC=N-) und Harnstoff-Gruppen (-NH(C=O)NH-). Bei den zur Knüpfung der jeweiligen kovalenten Bindung erforderlichen Reaktionen handelt es sich um Grundreaktionen der organischen Chemie, deren Reaktionsbedingungen dem Fachmann geläufig sind und den gängigen Nachschlagewerken (zum Beispiel Houben-Weyl, Methoden der Organischen Chemie, 4. Auflage) entnommen werden können.

Die zuvor als vorteilhaft beschriebenen Linker-Gruppen stellen eine nicht abschließende und lediglich beispielhafte Aufzählung von Möglichkeiten zur Knüpfung einer kovalenten Bindung zwischen dem Cyclodextrin und dem Polysaccharid dar. Grundsätzlich sind alle Arten von Linkern geeignet, durch die ein Cyclodextrin kovalent an ein Polysaccharid geknüpft werden kann.

Zur Herstellung der erfindungsgemäßen Verbindungen gemäß dieser ersten Ausführungsform müssen sowohl das Polysaccharid (PS) als auch das Cyclodextrin (CD) wenigstens eine freie funktionelle Gruppe aufweisen, die jeweils ausgewählt ist aus der Gruppe bestehend aus
- Hydroxy- (-OH) oder Mercapto-Gruppen (-SH);
- Carboxyl- oder Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
- Carbonsäurehalogenid-Gruppen (-CO-X, wobei X ein Halogenid ausgewählt aus Cl, Br und I ist);
- Amino-Gruppen (-NH₂);
- Aldehyd-Gruppen (-COH);
- Tosylat- (-SO₃C₇H₇), Mesylat (-SO₃CH₃), oder Triflat-Gruppen (-SO₃CF₃);
- Isocyanat-Gruppen (-NCO).

Zur Ausbildung der kovalenten Bindung ist es erforderlich, dass die freie funktionelle Gruppe des Cyclodextrins und die des Polysaccharids so gewählt werden, dass diese unter Bildung einer kovalenten Bindung miteinander reagieren können. Einen nicht abschließenden Überblick über die möglichen Kombinationen der beiden freien funktionellen Gruppen liefert die folgende Tabelle I, worin die freien funktionellen des PS und des CD gegeneinander ausgetauscht werden können.

| **Tabelle I** | | |
|---|---|---|
| Freie funktionelle Gruppe des Polysaccharids (PS) | Freie funktionelle Gruppe des Cyclodextrins (CD) | Resultierende Linker-Gruppe (L) |
| -OH/-SH | -(C=O)OR', -(C=O)-X | -(C=O)O-/ -(C=O)S- |
| -NH₂ | -(C=O)OR', -(C=O)-X | -NH-(C=O)- |
| -NCO | -OH | -NH-(C=O)O- |
| -NCO | -NH₂ | -NH-(C=O)-NH- |
| -NCO | -(C=O)OR' | -NH-(C=O)- |
| -(C=O)H | -NH₂ | -HC=N- |
| -(C=O)OR', -CO-X | -NCO | -NH-(C=O)- |

Die vorliegende Erfindung betrifft des Weiteren ein Verfahren zur Herstellung der Addukte gemäß der Formel I mit m = 0, durch Reaktion eines Polysaccharids (PS) mit einem Cyclodextrin (CD), wobei sowohl das Polysaccharid (PS) als auch das Cyclodextrin (CD) wenigstens eine freie funktionelle Gruppe aufweisen, die jeweils ausgewählt ist aus der Gruppe bestehend aus
- Hydroxy- (-OH) oder Mercapto-Gruppen (-SH);
- Carboxyl- oder Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
- Carbonsäurehalogenid-Gruppen (-CO-X, wobei X ein Halogenid ausgewählt aus Cl, Br und I ist);
- Amino-Gruppen (-NH₂);
- Aldehyd-Gruppen (-CHO)
- Isocyanat-Gruppen (-NCO);
wobei die Auswahl der freien funktionellen Gruppen des Cyclodextrins (CD) und des Polysaccharids (PS) wie folgt getroffen werden können:
(i) Weist das Cyclodextrin eine Hydroxy- (-OH) oder Mercapto-Gruppe (-SH) auf so kann das Polysaccharid eine freie funktionelle Gruppe aufweisen, die ausgewählt ist aus der Gruppe bestehend aus
   - Carboxyl- oder Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
   - Carbonsäurehalogenid-Gruppen (-CO-X, wobei X ein Halogenid ausgewählt aus Cl, Br und I ist);
   - Isocyanat-Gruppen (-NCO)
   um unter Bildung des kovalenten Linkers (L₁) miteinander zu reagieren. Alternativ können die miteinander reagierenden freien funktionellen Gruppen des Polysaccharids und des Cyclodextrins miteinander ausgetauscht werden.
ii) Weist das Cyclodextrin eine Amino-Gruppe (-NH₂) auf, so kann das Polysaccharid eine freie funktionelle Gruppe aufweisen, die ausgewählt ist aus der Gruppe bestehend aus
   - Carboxyl- oder Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
   - Carbonsäurehalogenid-Gruppen (-CO-X, wobei X ein Halogenid ausgewählt aus Cl, Br und I ist);
   - Isocyanat-Gruppen (-NCO);
   - Aldehyd-Gruppen (-CHO);
   um unter Bildung des kovalenten Linkers (L₁) miteinander zu reagieren. Alternativ können die miteinander reagierenden freien funktionellen Gruppen des Polysaccharid und des Cyclodextrin miteinander ausgetauscht werden.
(iii) Weist das Cyclodextrin eine Isocyanat-Gruppen (-NCO) auf, so kann das Polysaccharid eine freie funktionelle Gruppe aufweisen, die ausgewählt ist aus der Gruppe bestehend aus
   - Carboxyl-Gruppen;
   - Amino-Gruppen (-NH₂); und
   - Hydroxy- (-OH) oder Mercapto-Gruppen (-SH)
   um unter Bildung des kovalenten Linkers (L₁) miteinander zu reagieren. Alternativ können die miteinander reagierenden freien funktionellen Gruppen am Polysaccharid und am Cyclodextrin gegeneinander ausgetauscht werden.

### Bifunktionelle Linkermoleküle (m=1)

In einer weiteren erfindungsgemäßen Ausführungsform kann m = 1 sein. Der Linker (-L₁-R-L₂-) wird dann durch die Reaktion der zwei freien funktionellen Gruppen eines bifunktionellen Linkermoleküls, mit einer freien funktionellen Gruppe des Cyclodextrins einerseits, und mit einer weiteren freien funktionellen Gruppe des Polysaccharids andererseits, gebildet. Geeignete Linkermoleküle müssen zum Einen die notwendige Reaktivität zur selektiven Ausbildung der kovalenten Bindungen, zum Anderen jedoch auch eine ausreichende physiologische Verträglichkeit besitzen. Auch wenn die monomeren bifunktionellen Linkermoleküle aufgrund der notwendigen hohen Reaktivität eine damit verbundene Toxizität aufweisen, so ist entscheidend, dass diese in der kovalent gebundenen Form nicht mehr physiologisch bedenklich sind.

Geeignete bifunktionelle Linkermoleküle weisen zwei identische oder von einander verschiedene freie Funktionalitäten auf, die mit den freien funktionellen Gruppen des Cyclodextrins und des Polysaccharids jeweils unter Bildung einer kovalenten Bindung reagieren können. Die beiden funktionellen Gruppen sind durch eine Brückengruppe (R) von geeigneter Art und Länge kovalent miteinander verknüpft, wobei R wie oben definiert ist.

In einer Ausführungsform der vorliegenden Erfindung weist das bifunktionelle Linkermolekül zwei identische Funktionalitäten auf, die das Brückenmolekül (R) flankieren und die ausgewählt sind aus der Gruppe bestehend aus
- Hydroxy- (-OH) oder Mercapto-Gruppen (-SH);
- Carboxyl- oder Carbonsäureester-Gruppen (-COOR¹, wobei R¹ H oder eine C₁₋₁₀-Alkyl-Gruppe, vorzugsweise eine C₂₋₆-Alkyl-Gruppe, besonders bevorzugt C₃₋₅-Alkyl-Gruppe ist);
- Carbonsäurehalogenid-Gruppen (-CO-X, wobei X ein Halogenid ausgewählt aus Cl, Br und I ist);
- Amino-Gruppen (-NH₂);
- Aldehyd-Gruppen (-CHO);
- Isocyanat-Gruppen (-NCO).

Bevorzugte bifunktionelle Linkermoleküle mit identischen Funktionalitäten sind daher ausgewählt aus der Gruppe bestehend aus Dicarbonsäuren, Dicarbonsäureestern und Dicarbonsäureanhydriden, Dicarbonsäurehalogeniden, Diolen oder Dithiolen, Diaminen und Diisocyanaten, Dialdehyden und Phosgen.

Die Verwendung von bifunktionellen Linkermolekülen mit identischen Funktionalitäten trägt dazu bei, die Reaktion der bifunktionellen Molekülen miteinander zu unterdrücken.

Geeignete Dicarbonsäuren, Dicarbonsäureester, Dicarbonsäureanhydride, Dicarbonsäurehalogenide sind beispielsweise ausgewählt aus der Gruppe bestehend aus Oxalsäure, Malonsäure, Bernsteinsäure, Glutarsäure, Adipinsäure, Pimelinsäure, Azelainsäure, Sebacinsäure, Maleinsäure, Fumarsäure, Sorbinsäure, Phthalsäure, Terephthalsäure, Isophthalsäure, Agaricinsäure, Citronensäure, sowie deren C₁₋₁₀-Alkylestern, vorzugsweise C₂₋₆-Alkylestern, besonders bevorzugt C₃₋₅-Alkylestern, Halogeniden, und Anhydriden.

Geeignete Diisocyanate sind beispielsweise ausgewählt aus der Gruppe bestehend aus Toluylendiisocyanat, Bitoluylendiisocyanat, Dianisidindiisocyanat, Tetramethylendiisocyanat, Hexamethylendiisocyanat, m-Phenylendiisocyanat, m-Xylylendiisocyanat, C₁-C₆ Alkylbenzoldiisocyanat, 1-Chlorobenzol-2,4-diisocyanat, Cyclohexylmethandiisocyanat, 3,3'-Dimethoxydiphenylmethan-4,4'-diisocyanat, 1-Nitrobenzol-2,4-diisocyanat, 1-Alkoxybenzol-2,4-diisocyanat, Ethylendiisocyanat, Propylendiisocyanat, Cyclohexylen-1 ,2-diisocyanat, 3,3'-Dichloro-4,4'-biphenylendiisocyanat, Diphenylendiisocyanat, 2-Chlorotrimethylendiisocyanat, Butylen-1,2-diisocyanat, Ethylidendiisocyanat, Diphenylmethan-4,4'diisocyanat, Diphenylethandiisocyanat, 1,5-Naphthalindiisocyanat, Cyclohexandiisocyanat und Isophorondiisocyanat.

Geeignete Diole sind beispielsweise ausgewählt aus der Gruppe bestehend aus Ethylenglycol, Propylenglycol, Butylenglycol, und Neopentylglycol, Pentandiol-1,5,3-Methylpentandiol-1,5, Bisphenol A, 1,2- oder 1,4-Cyclohexandiol, Caprolactondiol (Reaktionsprodukt aus Caprolacton und Ethylenglycol), hydroxyalkylierte Bisphenole, Trimethylolpropan, Trimethylolethan, Pentaerythritol, Hexandiol-1,6, Heptandiol-1,7, Octandiol-1,8, Butandiol-1,4, 2-Methyloctandiol-1,8, Nonandiol-1,9, Decandiol-1,10, Cyclohexandimethylol, Di-, Tri- und Tetraethylenglykol, Di-, Tri- und Tetrapropylenglykol, Polyethylen- und Polypropylenglykole mit einem mittleren Molekulargewicht von 150 bis 15000, Trimethylolethan, Trimethylolpropan und Pentaerythrit.

Geeignete Diamine sind beispielsweise ausgewählt aus der Gruppe bestehend aus 1,2-Diaminoethan, 1,2- oder 1,3-Diaminopropan, 1,2-, 1,3- oder 1,4-Diaminobutan, 1,5-Diaminopentan, 2,2-Dimethyl-1,3-diaminopropan, Hexamethylendiamin, 1,7-Diamino-heptan, 1,8-Diaminooctan, Trimethyl-1,6-diaminohexan, 1,9-Diaminononan, 1,10-Diaminodecan, 1,12-Diaminododecan, 1,2-Diaminocyclohexan, 1,4-Diaminocyclohexan, 1,3-Cyclohexanbis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diaminopyridin, 2,3-Diaminopyridin, 3,4-Diaminopyridin, 2,4-Diaminopyrimidin, 4,5-Diaminopyrimidin, 4,6-Diaminopyrimidin.

In einer weiteren erfindungsgemäßen Ausführungsform weist das bifunktionelle Molekül zwei unterschiedliche funktionelle Gruppen auf, welche unabhängig voneinander ausgewählt sind aus der Gruppe bestehend aus
- Hydroxy- (-OH) oder Mercapto-Gruppen (-SH)-Gruppen (-OH);
- Carboxyl- und Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
- Carbonsäurehalogenid-Gruppen (-CO-X, wobei X ein Halogenid ausgewählt aus Cl, Br und I ist);
- Amino-Gruppen (-NH₂);
- Aldehyd-Gruppen (-CHO) und
- Isocyanat-Gruppen (-NCO).

Beispiele geeigneter bifunktioneller Linker-Moleküle mit unterschiedlichen Funktionalitäten sind ausgewählt aus der Gruppe bestehend aus
- Aminosäuren, sowie deren C₁₋₁₀-Alkylester und Halogenide ausgewählt aus Cl, Br und I;
- Hydroxycarbonsäuren oder Thiocarbonsäuren, ausgewählt aus der Gruppe bestehend aus α-, β- und γ-Hydroxy-/ Thiocarbonsäuren, wie beispielsweise Mandel-, Milch-, Äpfel-, Weinsäure, Ricinolsäure, Huminsäure, Salicylsäure (2-Hydroxybenzoesäure) und Gallussäure (3,4,5-Trihydroxybenzoesäure); und
- Aminoalkoholen, wie beispielsweise Aminoethanolen, Valinol und Leucinol.

Bei Verwendung von bifunktionellen Molekülen mit unterschiedlichen Funktionalitäten kann die Selektivität der Verknüpfung durch geeignete Schutzgruppen verbessert werden. Dabei wird zunächst eine der funktionellen Gruppen des bifunktionellen Moleküls blockiert während die andere freie funktionelle Gruppe mit dem Cyclodextrin oder dem Polysaccharid unter Ausbildung einer kovalenten Bindung reagieren kann. Im Anschluss daran wird die Schutzgruppe abgespalten, wodurch die zweite funktionelle Gruppe freigegeben wird und somit zur Knüpfung der zweiten kovalenten Bindung mit entweder dem Cyclodextrin oder dem Polysaccharid verwendet werden kann. Geeignete Schutzgruppen sind dem Fachmann aus beispielsweise aus Vogel's Textbook of Practical Organic Chemistry, 5. Aufl. S. 642 bekannt.

Beispielhafte Linkermoleküle zur Verknüpfung von SH- und NH-Funktionen sind ausgewählt aus der Gruppe bestehend aus: AMAS (N-α-(Maleimidoacetoxy)-succinimidester), BMPS (N-β-(Maleimidopropyloxy)succinimidester), GMBS (N-γ-(Maleimidobutyryloxy)succinimidester); EMCS (N-ε-(Maleimidocaproyloxy)succinimidester) MBS (m-(Maleimidobenzoyl)-N-hydroxysuccinimidester), SMCC (Succinimidyl4-(N-maleimidomethyl)cyclohexan-1-carboxylat), SMPB (Succinimidyl-4-(p-maleimidophenyl)butyrat), SPDP (Succinimidyl-3-(2-pyridyldithio)propionat), Sulfo-GMBS (N-γ-(Maleimidobutyryloxy)sulfosuccinimidester), Sulfo-EMCS (N-ε-(Maleimidocaproyloxy)sulfosuccinimidester).

Beispielhafte Linkermoleküle zur Verknüpfungvon SH- und SH-Funktionen sind ausgewählt aus der Gruppe bestehend aus BMB (1,4-Bismaleimidobutan), BMDB (1,4-Bis-maleimido-2,3-dihydroxybutan), BMH (Bis-maleimidohexan), BMOE (Bis-maleimidoethan), DTME (Dithio-bis-maleimidoethan), HBVS (1,6-Hexan-bis-vinylsulfon), BM(PEO)₃ (1,8-Bis-maleimidotriethylenglycol), BM(PEO)₄ (1,11-Bis-maleimidotetraethylenglycol).

Beispielhafte Linkermoleküle zur Verknüpfung von NH- und NH-Funktionen sind ausgewählt aus der Gruppe bestehend aus: BSOCOES (Bis (2(succinimidyloxycarbonyloxy)ethyl)sulfon, BS³ (Bis-(sulfosuccinimidyl)suberat), DFDNB (1,5-Difluor-2,4-dinitrobenzol), DMA (Dimethyladipimidat HCl), DSG (Disuccinimidylglutarat), DSS (Disuccinimidylsuberat), EGS (Ethylenglycol-bis-(succinimidylsuccinat).

Ein beispielhaftes Linkermolekül zur Verknüpfung von SH- und OH-Funktionen ist PMPI (N-(p-Maleimido-phenyl)isocyanat).

Beispielhafte Linkermoleküle zur Überführung einer SH-Funktion in eine COOH-Funktion sind ausgewählt aus der Gruppe bestehend aus BMPA (N-β-Maleimidopropionsäure), EMCH (N-β-Maleimidocapronsäure), KMUA (N-κ-Maleimidoundecansäure).

Ein beispielhaftes Linkermolekül, das mit einer Aminogruppe eines Moleküls reagiert und eine geschützte Amino-Gruppe in größerem Abstand von diesem Molekül zur Vermeidung einer sterischen Hinderung bereitstellt, ist ITCS (N-(Trifluoracetylcaproyloxy)succinimidester).

Weitere geeignete Linkermoleküle sind Fachleuten bekannt und im Handel erhältlich oder können je nach Bedarf und in Abhängigkeit von den vorhandenen und gewünschten funktionellen Gruppen der zukoppelnden Cyclodextrine und Kohlenhydrate entworfen und nach bekannten Verfahren hergestellt werden.

Wenn der Linker (-L₁-R-L₂-) unter Verwendung eines bifunktionellen Linkermoleküls gebildet wird, sind sowohl die freien funktionellen Gruppen des Cyclodextrins, des Polysaccharids als auch die des bifunktionellen Moleküls so zu wählen, dass diese miteinander reagieren können.

Einen nichtabschließenden Überblick über die möglichen Kombinationen der freien funktionellen Gruppen liefert die folgende Tabelle II.

| **Tabelle II** | | | |
|---|---|---|---|
| (CD) | (PS) | bifunktionelles Linkermolekül | Addukt |
| -OH/-SH | -OH/-SH | R-(NCO)₂, R-(COOR¹)₂ R-(CO-X)₂ Cl-(C=O)-Cl | (CD)-O(C=O)HN-R-NH-(C=O)O-(PS)/ (CD)-S(C=O)HN-R-NH-(C=O)S-(PS) (CD)-O(O=C)-R-(C=O)O-(PS)/ (CD)-S(O=C)-R-(C=O)S-(PS) CD-S-(O=C)-S-PS/ CD-O-(O=C)-O-PS / CD-S-(O=C)-O-PS / CD-O-(O=C)-S-PS |
| -COOR¹ -CO-X | -COOR¹ -CO-X | R-(NH₂)₂, R-(OH)₂/R-(SH)₂ R-(NCO)₂, | (CD)-(C=O)HN-R-NH-(C=O)-(PS) (CD)-(C=O)-O-R-O-(O=C)-(PS)/ (CD)-(C=O)-S-R-S-(O=C)-(PS) (CD)-(C=O)-HN-R-NH-(C=O)-(PS) |
| -NH₂ | -NH₂ | R-(COOR¹)₂ R-(NCO)₂ Cl-(C=O)-Cl R-(CHO)₂ | (CD)-HN-(O=C)-R-(C=O)-NH-(PS) (CD)-HN-(O=C)-HN-R-NH-(C=O)-NH-(PS) (CD)-HN-(O=C)- NH-(PS) (CD)-HN=CH-R-CH=NH-(PS) |
| -NCO | -NCO | R-(NH₂)₂, R-(OH)₂/ R-(SH)₂, R-(COOR¹)₂ | (CD)-HN(O=C)HN-R-NH(C=O)NH-(PS) (CD)-HN(O=C)O-R-O(C=O)NH-(PS)/ (CD)-HN(O=C)S-R-S(C=O)NH-(PS) (CD)-HN(O=C)-R-(C=O)NH-(PS) |
| -COOR¹ -CO-X | -OH/-SH | H₂N-R-COOR¹ oder H₂N-R-CO-X | (CD)-(C=O)NH-R-(C=O)O-(PS) |
| | | HO-R-NCO/ HS-R-NCO | (CD)-(C=O)O-R-NH-(C=O)O-(PS)/ (CD)-(C=O)O-R-NH-(C=O)S-(PS) |
| | | H₂N-R-NCO | (CD)-(C=O)-NH-R-NH-(C=O)O-(PS) |
| | | OCN-R-COOR¹, | (CD)-(C=O)HN-R-(C=O)O-(PS)' |
| | | | |
| -NH₂ | -OH/ -SH | ROOC-R-NCO, | (CD)-NH(O=C)-R-NH-(C=O)O-(PS)/ (CD)-NH(O=C)-R-NH-(C=O)S-(PS), |
| | | R-(COOR¹)₂ oder R-(CO-X)₂ | (CD)-NH(O=C)-R-(C=O)O-(PS) |
| | | R-(NCO)₂ | (CD)-NH-(O=C)-NH-R-NH-(C=O)O-(PS) |
| -NCO | -OH/ -SH | H₂N-R-COOH | (CD)-NH-(C=O)-NH-R-(C=O)O-(PS)/ (CD)-NH-(C=O)-NH-R-(C=O)S-(PS) |
| -COOR¹, -CO-X | -NH₂ | HO-R-COOH/ HS-R-COOH | (CD)-(C=O)O-R-(C=O)NH-(PS)/ (CD)-(C=O)S-R-(C=O)NH-(PS) |
| | | H₂N-R-COOH | (CD)-(C=O)NH-R-(C=O)NH-(PS) |
| | | HO-R-NCO/ HS-R-NCO | (CD)-(C=O)O-R-NH(C=O)NH-(PS) (CD)-(C=O)S-R-NH(C=O)NH-(PS) |

In einer Ausführungsform der vorliegenden Erfindung sind die freien funktionellen Gruppen des Polysaccharids (PS) und des Cyclodextrins (CD) identisch.

Die vorliegende Erfindung betrifft daher ein Verfahren zur Herstellung der Addukte gemäß der Formel I mit m=1, durch Reaktion eines Polysaccharids (PS) und eines Cyclodextrins (CD) mit einem bifunktionellen Molekül unter Bildung des Linkers (-L₁-R-L₂-).
(i) In einer ersten Ausführungsform dieses erfindungsgemäßen Verfahrens weisen sowohl das Polysaccharid (PS) als auch das Cyclodextrin (CD) wenigstens eine freie funktionelle Gruppe auf, die unabhängig voneinander ausgewählt sind aus der Gruppe bestehend aus
   - Carboxyl- oder Carbonsäureester-Gruppen (-COOR¹, wobei R¹ Wasserstoff oder eine C₁₋₁₀-Alkyl-Gruppe, vorzugsweise eine C₂₋₆-Alkyl-Gruppe, besonders bevorzugt C₃₋₅-Alkyl-Gruppe ist);
   - Carbonsäurehalogenid-Gruppen (-CO-X, wobei X ein Halogenid ausgewählt aus Cl, Br und I ist);
   - Isocyanat-Gruppen (-NCO).
   Diese freien funktionellen Gruppen am Cyclodextrinen und Polysacchariden können mit einem Diamin der allgemeinen Formel III

   R(-NH₂)₂ (III);

   mit einem Diol der allgemeinen Formel IV

   R¹(-OH)₂ (IV);

   mit einem Dithiol der allgemeinen Formel V

   R¹(-SH)₂ (V);

   mit einem Aminoalkohol der allgemeinen Formel (VI)

   H₂N-R-OH (VI);

   mit einem Mercaptoalkohol der allgemeinen Formel (VII)

   HS-R-OH (VII);

   unter Ausbildung des kovalenten Linkers (-L₁-R-L₂-) zum erfindungsgemäßen Addukt reagieren.
(ii) In einer weiteren alternativen Ausführungsform des erfindungsgemäßen Verfahrens weisen sowohl das Polysaccharid (PS) als auch das Cyclodextrin (CD) jeweils wenigstens eine freie funktionelle Gruppe auf, die unabhängig voneinander ausgewählt sind aus der Gruppe bestehend aus
   - Isocyanat-Gruppen (-NCO);
   - Amino-Gruppen (-NH₂); und
   - Hydroxy- (-OH) oder Mercapto-Gruppen (-SH).
   Diese freien funktionellen Gruppen am CD und PS können mit einer Dicarbonylverbindung, die ausgewählt ist aus der Gruppe bestehend aus
   - Dicarbonsäuren oder Diestern der allgemeinen Formel VIII

      R-(COOR¹)₂ (VIII),

      wobei R¹ Wasserstoff oder eine C₁₋₁₀-Alkylgruppe ist;
   - Dicarbonsäurehalogeniden der allgemeinen Formel IX

      R (-CO-X)₂ (IX);
   - Dicarbonsäureanhydriden der allgemeinen Formel (VIII)

      R-(CO)₂O (X);
   - Dialdehyden der allgemeinen Formel (XI)

      R-(CHO)₂ (XI);

      unter Ausbildung des kovalenten Linkers (-L₁-R-L₂-) zum erfindungsgemäßen Addukt reagieren.
(iii) In einer weiteren alternativen Ausführungsform des erfindungsgemäßen Verfahrens weisen sowohl das Polysaccharid (PS) als auch das Cyclodextrin (CD) wenigstens eine freie funktionelle Gruppe auf, die unabhängig voneinander ausgewählt sind aus der Gruppe bestehend aus
   - Carboxyl-Gruppen (-COOH);
   - Hydroxy- (-OH) oder Mercapto-Gruppen (-SH);
   - Amino-Gruppen (-NH₂).
   Diese freien funktionellen Gruppen am Cyclodextrin und Polysaccharide können mit einem Diisocyanat der allgemeinen Formel XII

   R(-NCO)₂ (XII),

   unter Bildung des Linkers (-L₁-R-L₂-) reagieren.
(iv) In einer weiteren alternativen Ausführungsform des erfindungsgemäßen Verfahrens weisen sowohl das Polysaccharid (PS) als auch das Cyclodextrin (CD) jeweils wenigstens eine freie funktionelle Gruppe auf, die unabhängig voneinander ausgewählt sind aus der Gruppe bestehend aus
   - Amino-Gruppen (-NH₂); und
   - Hydroxy- (-OH) oder Mercapto-Gruppen (-SH).

Diese freien funktionellen Gruppen am Cyclodextrin und Polysaccharid können mit Phosgen (Cl₂(C=O)) unter Bildung des Linkers (-L₁-R-L₂-) reagieren.

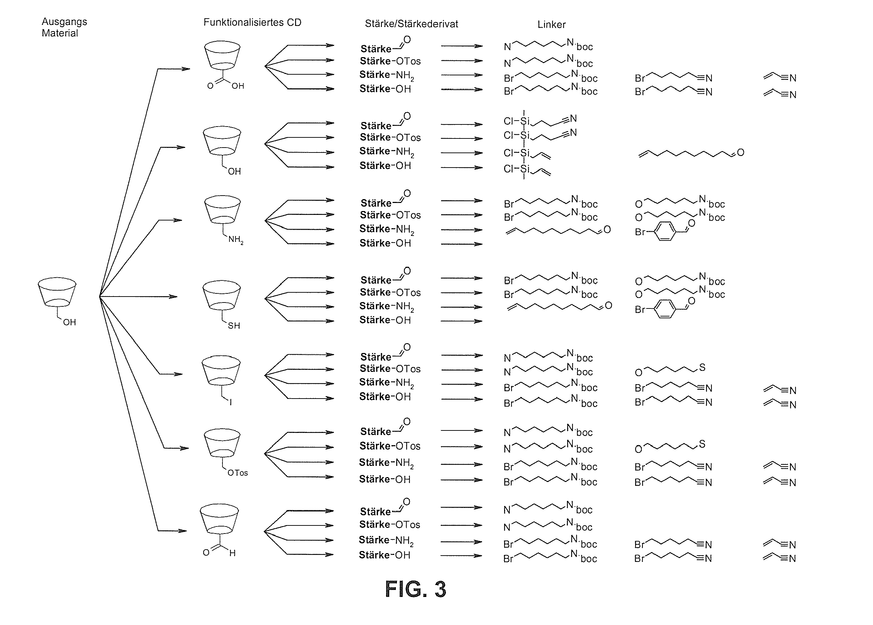

Weitere geeignete Linker und deren Kombinationen mit Cyclodextrinen und Polysacchariden sind in Figur 3 beschrieben.

Besonders bevorzugt gemäß der vorliegenden Erfindung sind die Addukte gemäß den nachfolgenden Formeln XIII bis XXIV:

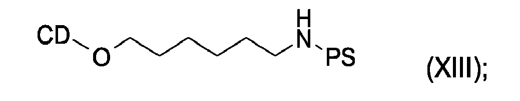

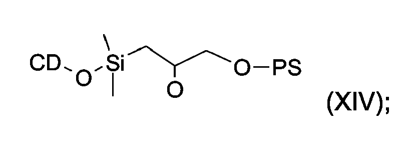

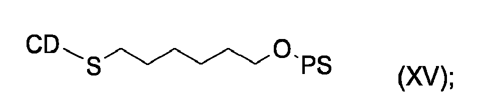

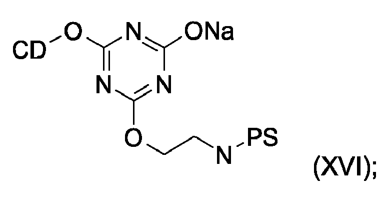

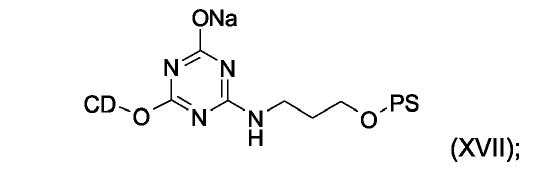

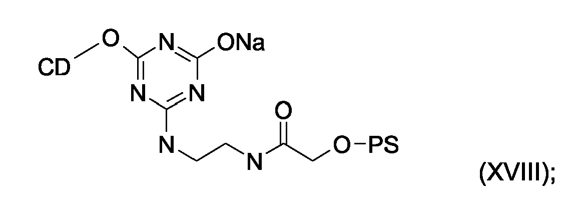

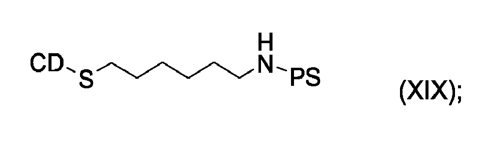

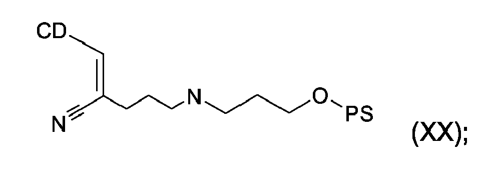

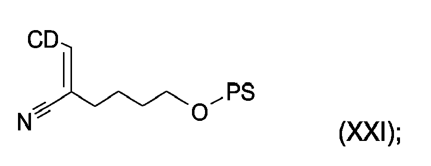

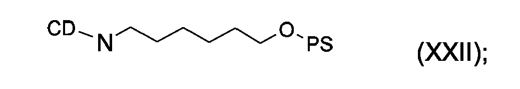

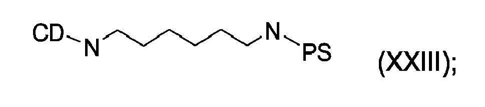

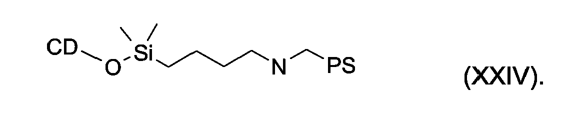

### Addukt/Wirkstoff Komplexe

Die zuvor beschriebenen erfindungsgemäßen Addukte können als Bestandteil von pharmazeutischen Zusammensetzungen verwendet werden. In der Regel weisen solche Zusammensetzungen wenigstens einen physiologisch, vorzugsweise arzneilich wirksamen Bestandteil auf, der mit den erfindungsgemäßen Addukten in Wechselwirkung treten kann. Insbesondere handelt es sich dabei um Wirkstoffe mit einer geringen Wasserlöslichkeit, die durch Komplexierung mit den erfindungsgemäßen Addukten solubilisiert werden.

Die Erfindung betrifft daher zudem einen Addukt/Wirkstoff Komplex umfassend eines der zuvor beschriebenen Addukte und einen pharmazeutischen Wirkstoff.

Bevorzugte pharmazeutische Wirkstoffe sind beispielhaft ausgewählt aus der Gruppe bestehend aus Hormonen, insbesondere Steroidhormonen (z.B. Progesteron), Cytostatika, Antibiotika (z.B. Gentamycin), Fungiziden (z.B. Amphotericin B), Allgemeinanästhetika (z.B. Propofol), Hypnotika (z.B. Diazepam), Analgetika (z.B. Fentanyl) und Immunsuppressiva (z.B. Cyclosporin A). Im Verhältnis zum komplexierenden Wirkstoff werden die erfindungsgemäßen Addukte vorzugsweise im Überschuss eingesetzt. Erfindungsgemäß können MolVerhältnisse (Cyclodextrin-Einheit : Wirkstoff) von 1000:1 vorzugsweise 100:1, besonders bevorzugt von 10:1 eingesetzt werden.

Die erfindungsgemäßen pharmazeutischen Zusammensetzungen umfassen die erfindungsgemäßen Addukte in einer Menge von 0,1 bis 50 Gew.-%, vorzugsweise von 0,1 bis 30 Gew.-%, besonders bevorzugt von 10 bis 20 Gew.-%, bezogen auf die Gesamtzusammensetzung.

In einer bevorzugten Ausführungsform liegen die erfindungsgemäßen Addukte in der pharmazeutischen Zusammensetzungen in Form einer wässrigen Lösung vor.

Die Erfindung soll anhand der folgenden Ausführungsbeispiele näher erläutert werden. Es ist jedoch nicht beabsichtigt den Gegenstand der vorliegenden Anmeldung auf diese Beispiele zu beschränken.

### Beispiele

### Beispiel 1 : Synthese von Carboxymethylstärke (CMS ; MS : 0.3 ; Mw: 60 kDa)

Dünnkochende Wachsmaisstärke (Cerestar®) wurde in einem wässrig-alkalischen Medium mit Chloressigsäure im molaren Verhältnis Stärke (Anhydro-Glucose) : Chloressigsäure von 1:4 bei 40°C für 2 Stunden umgesetzt. Durch anschließende saure Hydrolyse mit HCl (pH : 1,8 / 90°C) wurde das Mw (Gewichtsmittel) des Polymers auf 55 kDa reduziert und mittels Dialyse unter Verwendung von Dialysierschläuchen z.B. MWCO 1000 (Roth, Deutschland) für 24 Stunden gegen Wasser bei Raumtemperatur aufgereinigt. Die Überwachung des hydrolytischen CMS-Abbau sowie die Molekulargewichtsbestimmung des Endproduktes erfolgte dabei mittels GPC-MALLS (RI- Detektor: Optilab DSP / MALLS- Detektor: EOS / Software: Astra; Wyatt Technology, Woldert, Deutschland) unter Verwendung von 4 sequenziellen TCKGel Säulen (G 6000 PW / G 5000 PW, G 3000 PW / G 2000 PW ; Tosoh Biosep, Japan) in 50 mM Phosphatpuffer pH 7,0 bei einer Flussrate von 0,8 ml/Minute quantifiziert (dn/dc : 0,144). Die CMS wurde anschließend im Rotationsverdampfer bei 90°C getrocknet. Das Mw nach der Dialyse betrug 58 kDa. Zur Bestimmung des molaren Substitutionsgrades wurde die CMS in schwefelsaurer Lösung hydrolysiert und die 2-Hydroxyessigsäure bei 540 nm spektralphotometrisch quantifiziert. Der molare Substitutionsgrad (MS) betrug 0,31. Diese Substanz diente als Kopplungskomponente für die Lysin-derivatisierten carboxymethyl-Cyclodextrine (s.u.).

### Beispiel 2 : Synthese von mono-carboxymethyliertem 2-HP-β-CD

Die Carboxymethylierung von Cyclodextrinen ist am Beispiel der Synthese einer carboxymethylierten 2-HP-β-CD beispielhaft ausgeführt. Eine 10%ige wässrige Lösung von 2-HP-β-CD (Cleptose® HPB, Roquette, Frankreich) wurde mit Chloressigsäure im alkalischen Milieu im molaren Verhältnis 2-HP-β-CD : Chloressigsäure 1,5 : 1 für 2 Stunden bei 40°C umgesetzt. Nach 24 stündiger Dialyse gegen Wasser unter Verwendung von Dialysierschläuchen z.B. MWCO 500 (Roth, Deutschland) wurde das carboxylierte CD- Derivat mittels präparativer Anionenaustausch- Chromatographie unter Verwendung des Austauscherharzes Dowex® 1 (Fluka, Deutschland) vom nicht umgesetzten Cyclodextrin abgetrennt. Die an den Ionenaustauscher gebundenen carboxymethylierten Cyclodextrin-Derivate wurden anschließend mit 10%iger NaCl- Lösung eluiert und durch erneute Dialyse wie oben beschrieben entsalzt und unter Vacuum getrocknet. Unter vergleichbaren Reaktionsbedingungen wurden die hauptsächlich monocarboxymethylierten Derivate von α-, β-, γ- CD hergestellt.

### Beispiel 3: Kopplung von carboxymethyliertem 2-HP-β-Cyclodextrin an Carboxymethylstärke

Das CM-CD aus Beispiel 2 wurden anschließend mit Nα-BOC-L-Lysin (Sigma, Deutschland) wie folgt umgesetzt. In einem Rundkolben mit Magnetrührer wurden 6 g des CM-CD und der 4-fachen molaren Überschuss des Lysin- Derivates in CH₂Cl₂ vollständig gelöst. Im zweistunden- Abstand wurden jeweils viermal 1 g N-(3-Dimethylaminopropyl)-N'-ethylcarbodiimid (EDC), gelöst in CH₂Cl₂, zugegeben und der Reaktionsansatz anschließend über Nacht weitergerührt. Die Amid-Kopplung der CM-CD erfolgte an der ε- Amino-Gruppe des Lysins. Nach Abspaltung der Schutzgruppe in Gegenwart von Ameisensäure wurde dieses Derivat an der nun freien α-Aminogruppe mit Carboxymethylstärke CMS (MS : 0,3; Mw: 60 kDa) wie oben beschrieben im molaren Verhältnis Lys-CD: unsubstituierter/ carboxymethylierter Anhydroglucose- Einheit von CMS von 2 :1 umgesetzt und erneut gegen Wasser dialysiert. Das Molekulargewicht des Konstruktes betrug 195 kDa. Unter vergleichbaren Reaktionsbedingungen wurden die Lysin- Derivate der in Beispiel 2 beschriebenen carboxymethylierten α-, β-, γ-CD- Derivate synthetisiert.

### Beispiel 4: Bestimmung des prozentualen Anteils des Cyclodextrins am Addukt

Die Bestimmung des prozentualen Anteils des CD am Addukt der gemäss Beispiel 3 hergestellten Cyclodextrin-Derivate erfolgte nach alkalischer Hydrolyse durch Quantifizierung der freigesetzten Cyclodextrine mittels GPC-MALLS und ist nachfolgend am Beispiel des β-CD-Konstruktes näher erläutert. 1 g des Cyclodextrin- Derivates wurde in 10 ml Wasser aufgenommen, mit 5 ml 10%iger KOH versetzt und im kochenden Wasserbad 3 Stunden hydrolysiert. Das Reaktionsgemisch wurde anschließend neutralisiert, durch 0,45 µm Filter filtriert und mittels GPC-MALLS (RI- Detektor: Optilab DSP / MALLS- Detektor: EOS / Software : Astra ; Wyatt Technology, Woldert, Deutschland) unter Verwendung von 4 sequenziellen TCKGel Säulen (G 6000 PW / G 5000 PW, G 3000 PW / G 2000 PW ; Tosoh Biosep, Japan) in 50 mM Phosphatpuffer pH 7,0 bei einer Flussrate von 0,8 ml/Minute quantifiziert (dn/dc : 0,144). Der Cyclodextrin- Anteil am Konstrukt betrug 61,5% (w/w).

### Beispiel 5 : Vergleich der Hämolyserate unterschiedlicher Addukte

Die Hämolyserate der in Tabelle III aufgeführten Cyclodextrin-Derivate wurde wie folgt bestimmt. Humane Erythrozyten wurden aus Citratblut durch Zentrifugation gewonnen (1000 x g / 10 Minuten), in 10 mM isotonem Phosphatpuffer (pH 7,4) aufgenommen und dreimal gewaschen und in diesem Puffer auf einen Hämatokrit von 5% eingestellt. Aliquote der gewaschenen Erythrozyten (0,1 ml) wurden zu 4 ml der in der Tabelle 3 aufgelisteten Cyclodextrin-Derivate (jeweils 5 unterschiedliche Konzentrationen) zugegeben, gemischt und 30 Minuten bei 37°C und gelegentlichem leichten Schütteln inkubiert. Nach abermaliger Zentrifugation (1000 x g / 10 Minuten) wurde das freigesetzten Hämoglobin im Überstand spektralphotometrisch bei 543 nm bestimmt. Die Hämolyseraten wurde aus den erhaltenen Absoptionswerten im Vergleich mit einer kompletter Hydrolyse in Wasser ermittelt und die Cyclodextrin- Konzentrationen mit halbmaximaler Hämolyse graphisch ermittelt.

**Tabelle III : Hämolyse gewaschener menschlicher Erythrozyten (0,25%) in 10 mM isotonem Phosphatpuffer (pH 7,4) bei 37°C/30 Minuten durch Cyclodextrin-Derivate**

| **Tabelle III** | |
|---|---|
| Cyclodextrin- Derivat (hergestellt gemäß Beispielen 1 bis 3) | Cyclodextrin-Konzentration (frei bzw.gebunden) zur halb-maximalen Hämolyse [mM] |
| α-Cyclodextrin | 11 |
| α-Cyclodextrin-PS-Addukt | > 100 |
| β-Cyclodextrin | 5 |
| β-Cyclodextrin-PS-Addukt | > 50 |
| 2-HP-β-Cyclodextrin | 10 |
| 2-HP-β-Cyclodextrin-PS-Addukt | > 100 |
| γ--Cyclodextrin | 30 |
| γ--Cyclodextrin-PS-Addukt | > 100 |

### Beispiel 6 : Vergleich der Löslichkeiten von Addukten und freien CD

Die lösungsvermittelnden Eigenschaften einer Reihe von freien Cyclodextrine sowie deren Kolloid-gekoppelten Derivate wurden wie folgt bestimmt. Zu den jeweiligen Lösung der entsprechenden Cyclodextrine oder deren Derivate wurde ein Überschuss der in Tabelle IV aufgeführten Arzneistoffe gegeben, in Glasvials bei 121°C für 20 Minuten hitzebehandelt und zur Gleichgewichtseinstellung 3 Tage bei Raumtemperatur gelagert. Anschließend wurden die Suspensionen durch 0,45 µm Filter filtriert und die Wirkstoff-Konzentration im Filtrat spektralphotometrisch bestimmt.

**Tabelle IV : Lösungsvermittelnde Eigenschaften von Cyclodextrinen und ihren Derivaten**

| **Tabelle IV** | | |
|---|---|---|
| CD-Derivat (hergestellt gemäß Beispielen 1 bis 3) | Arzneistoff | Löslichkeit Arzneistoff [g/l] |
| 2-HP-β-CD 40% (w/v) | Progesteron | 34 |
| 2-HP-β-CD-PS-Addukt 40% (w/v) | Progesteron | 33 |
| γ-CD 10%(w/v) | Progesteron | 0,61 |
| γ-CD-PS-Addukt 10% (w/v) | Progesteron | 0,60 |
| 2-HP-β-CD 15% (w/v) | Megestrol-Acetat | 0,64 |
| 2-HP-β-CD-PS-Addukt 15% (w/v) | Megestrol-Acetat | 0,64 |
| γ-CD 15% (w/v) | Megestrol-Acetat | 0,53 |
| γ-CD-PS-Addukt 15% (w/v) | Megestrol-Acetat | 0,52 |

Wie aus Tabelle IV ersichtlich besitzen die Addukte in gleichen Konzentrationen etwa vergleichbare lösungsvermittelnde Eigenschaften; wie die Ausgangs-Cyclodextrine in wässrigen Lösungen. Die Löslichkeiten der beiden Arzneistoffe in Wasser wurde auf gleiche Weise bestimmt und betrug 15 mg/l für Progesteron und 0,6 mg/l für Megastrol-Acetat.

### Beispiel 7: (CD)-O-N-Stärke

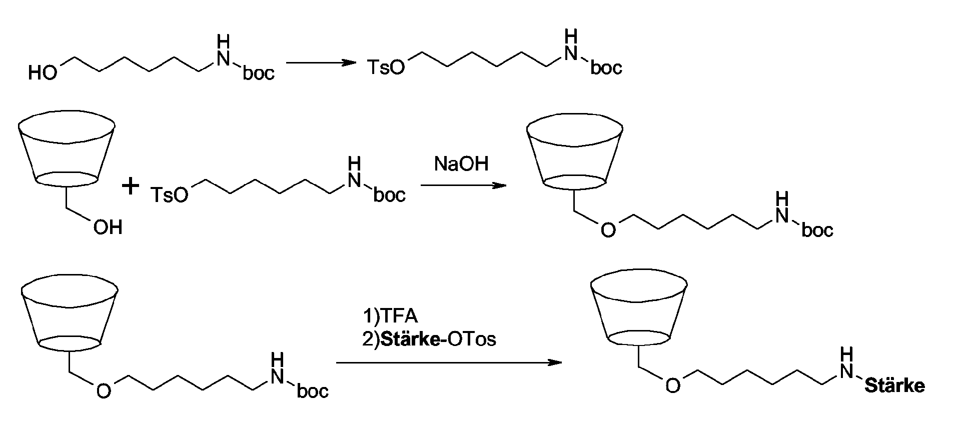

1. Stärke-O- + Br-Alkyl: Annis I., Chen L., Barany G., J. Am. Chem. Soc. 1998 120 7226-38;
2. Tosylierung von Cellulose: US-A-4286958 S. 11-12 Beispiel 10;
3. Tosylierung von Stärke: JOC 1963 28 3553.
Verwendete Reagenzien: 6-(Boc-amino)-1-hexanol, Tosylchlorid.

### Beispiel 8: CD-OSi-O-Stärke

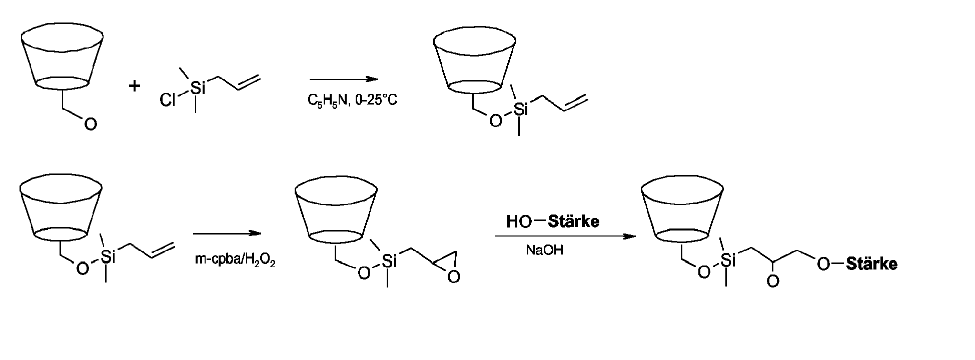

1. Monosilylierung "Definiert Monofunktionalisierte β-Cyclodextrinderivate' H.Dittmann S.196;
2. Silylierung primärer Alcohole: Ashton P.R., Königer R., Stoddart J.F., Alker D., Harding V.D. J. Org. Chem. 1996 61 903-908;
3. Stärke end Epoxide: Ullman's encyclopedia of industrial chemistry, Vol A25 S. 19;
4. Hydroxyethylstärke: H.Merkus, J.Mourits, L.deGalan, W.deJong, Stärke, 1977 (29) 406.
Verwendete Reagenzien: Allyl(chlor)dimethylsilan, 3-Chloroperbenzoesäure (m-cpba)

### Beispiel 9: CD-O-N-Stärke

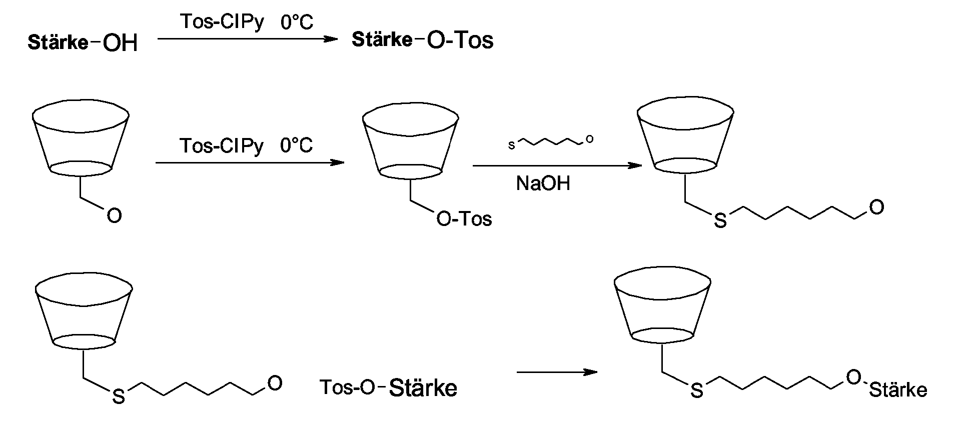

1. Tosylierung von Cellulose: US-A-4286958 S. 11-12 Beispiel 10;
2. Tosylierung von Stärke: JOC 1963 28 3553;
3. Monotosylierung von Cyclodextrinen: L.D. Melton and K.N. Slessor, Carbohydr. Res., 1971 (18) 29-33;
4. Thiocyclodextrine: Griffith D.W., Bender M.L., Adv.Cat., 1973, 54, 625; B.Siegel J.Inorg. Nucl.Chem. 1979 41(4), 609-610.
Verwendete Reagenzien: 6-Mercapto-1-hexanol, Tosylchlorid.

### Beispiel 10: CD-O-N-Stärke

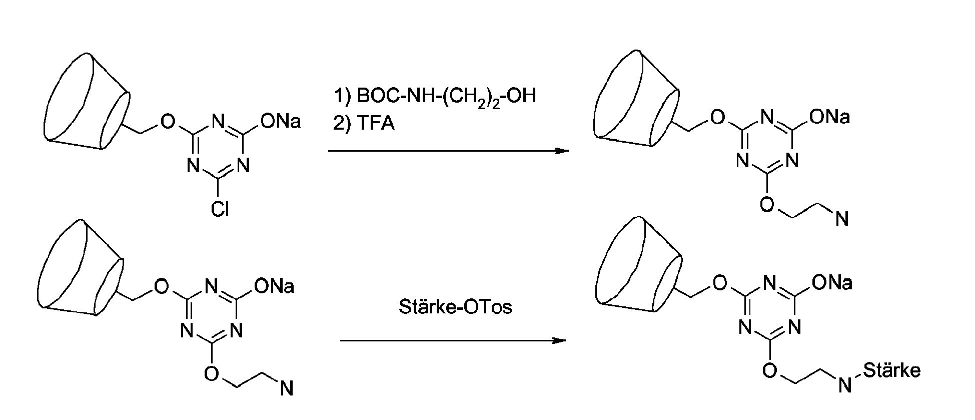

1. Monochlortriazinyl-β-cyclodextrin (MCT-CD) Reaktionen: Stieger G., 'Covalent and supramolecularinteraction of ceramic particles', Report 129, 2002 Universität Stuttgart S. 58-59;
2. Tosylierung von Cellulose: US-A-4286958 S. 11-12 Beispiel 10;
3. Tosylierung von Stärke: JOC 1963 28 3553.
Verwendete Reagenzien: MCT-β-Cyclodextrin, N-Boc-Ethanolamin, Tosylchlorid.

### Beispiel 11: CD-O-N-Stärke

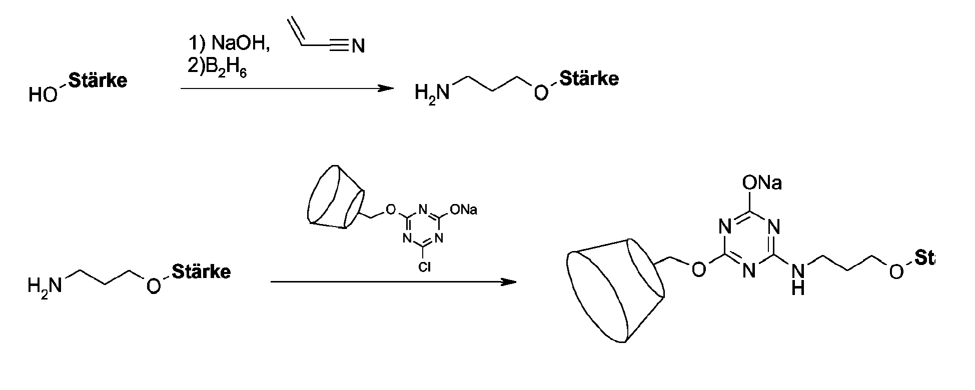

1. Organische Synthese an der Festphase, Supports for Solid-Phase Organic Synthesis S. 31;
2. Cyanoethylierung von Cellulose: Englebretsen, D.R.; Harding, D.R.K. Int. J. Pept. Prot. Res., 1992, 40, 487-496;
3. Monochlortriazinyl-β-cyclodextrin (MCT-CD) Reaktionen: Stieger G., 'Covalent and supramolecularinteraction of ceramic particles', Report 129, 2002 Universität Stuttgart S. 58-59.
Verwendete Reagenzien: Acrylnitril, Diboran, MCT-β-Cyclodextrin.

### Beispiel 12: CD-O-NC(O)-O-Stärke

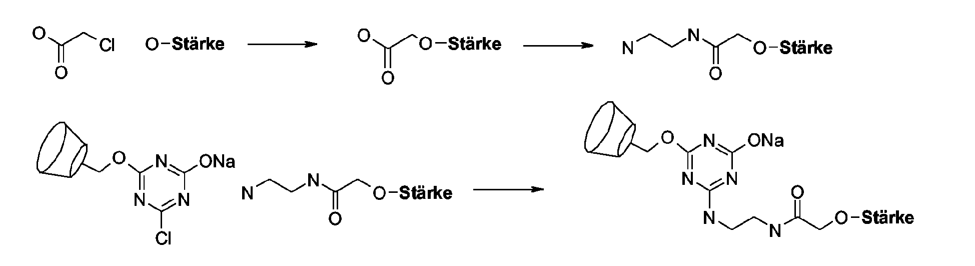

1. Monochlortriazinyl-β-cyclodextrin (MCT-CD) Reaktionen: Stieger G., 'Covalent and supramolecularinteraction of ceramic particles', Report 129, 2002 Universität Stuttgart S. 58-59;
2. Carboxymethylation of dextran: Makoto Hattori,* Koichi Nagasawa, Koki Ohgata, Noriko Sone, Ayumu Fukuda, Hiroshi Matsuda, and Koji Takahashi Bioconjugate Chem., 11 (1), 84-93, 2000.
Verwendete Reagenzien: MCT-β-Cyclodextrin, Chloressigsäure, Ethylendiamin.

### Beispiel 13: CD-N-N-Stärke

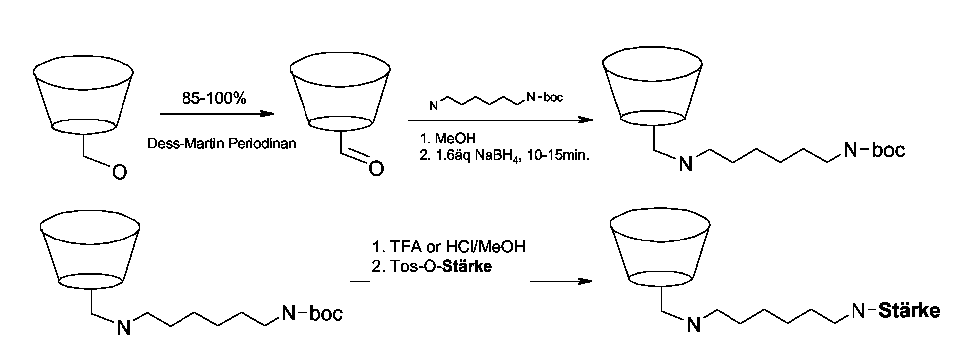

1. Monosilylierung: 'Definiert monofunktionalisierte β-Cyclodextrinderivate' H.Dittmann S.196;
2. Silylierung primärer Alkohole: Ashton P.R., Königer R., Stoddart J.F., Alker D., Harding V.D. J. Org. Chem. 1996 61 903-908;
3. Stärke und Epoxide: Ullman's encyclopedia of industrial chemistry, Vol A25 S 19;
4. Hydroxyethylstärke: H.Merkus, J.Mourits, L.deGalan, W.deJong, Stärke, 1977 (29) 406.
Verwendete Reagenzien: Allyl(chlor)dimethylsilane, 3-Chlorperoxybenzoesäure.

### Beispiel 14: (CD)-S-N-Stärke

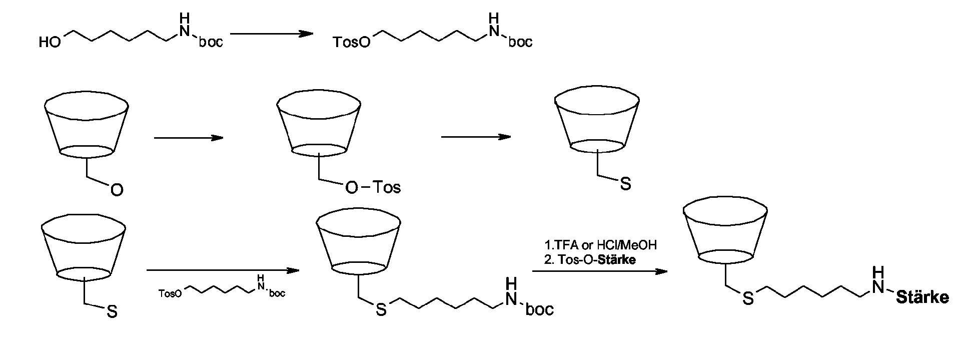

1. Monotosylierung von Cyclodextrinen: L.D. Melton and K.N. Slessor, Carbohydr. Res., 1971 (18) 29-33;
2. Mercaptocyclodextrin: Macromol. Chem. Phys. 1994 195 1719-1732;
3. Tosylierung von Cellulose: US-A-4286958 S. 11-12 Beispiel 10;
4. Tosylierung von Stärke: JOC 1963 28 3553.
Verwendete Reagenzien: 6-Brom-1-hexanol, Tosylchlorid.

### Beispiel 15: (CD)-C-N-O-Stärke

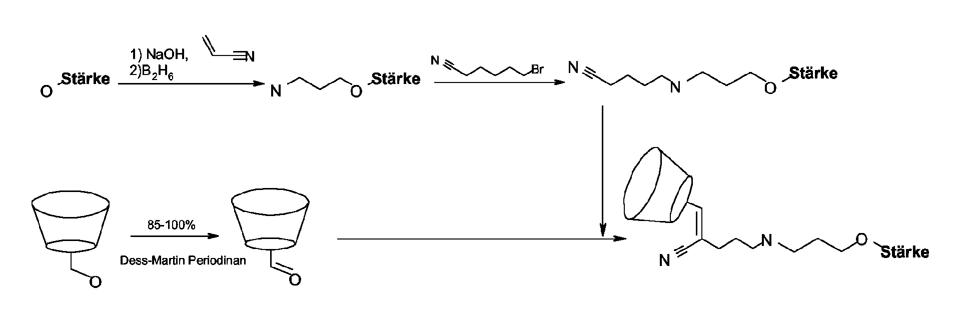

1. Englebretsen, D.R.; Harding, D.R.K. Int. J. Pept. Prot. Res., 1992, 40, 487-496;
2. Cyclodextrinmonoaldehyd: M. J. Cornwell J. B. Huff C. Bieniarz Tet Lett 1995 36(46) 8371-8374;
Verwendete Reagenzien: 6-Bromhexannitril, Acrylnitril, Diboran, DMP (Dess-Martin Periodinan).

### Beispiel 16: (CD)-O-N-Stärke

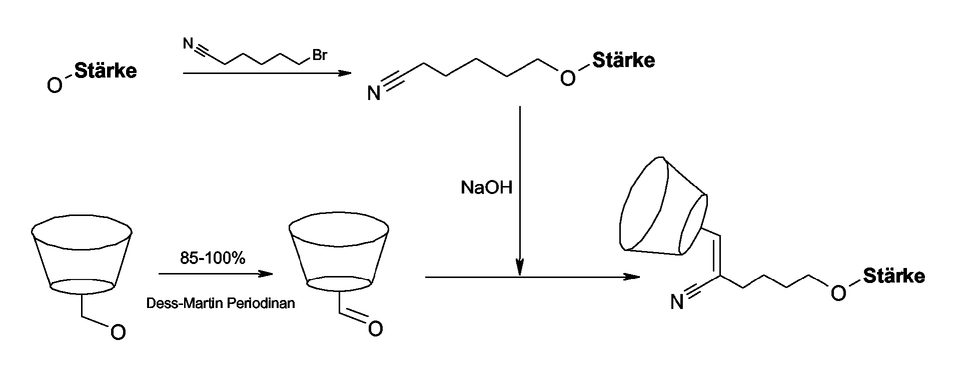

1. Cyclodextrinmonoaldehyd: M. J. Cornwell, J. B. Huff, C. Bieniarz, Tet. Lett. 1995 36(46), 8371-8374;
2. Stärke-O-alkyl via Brom: Carbohydrate Research 337 (2002) 2263-2272.
Verwendete Reagenzien: 6-Bromhexannitril, DMP (Dess-Martin Periodinan).

### Beispiel 17: (CD)-N-O-Stärke

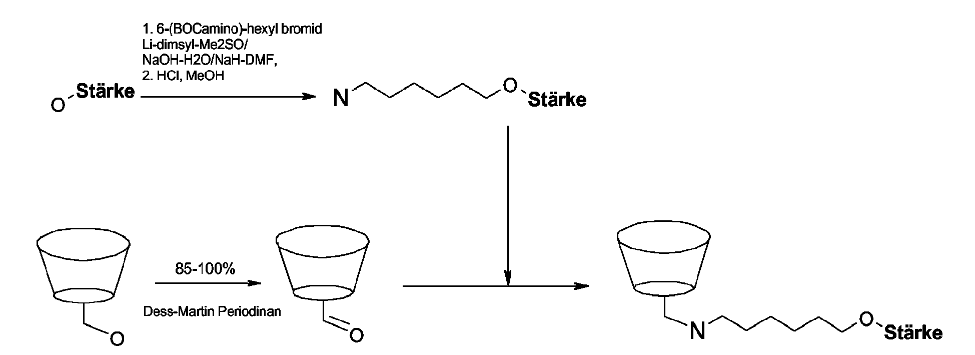

1. Stärke-O-alkyl via Brom: Carbohydrate Research 337 (2002) 2263-2272;
2. Cyclodextrinmonoaldehyd: M. J. Cornwell, J. B. Huff, C. Bieniarz, Tet. Lett. 1995 36(46) 8371-8374;
3. Reduktive Aminierung: A. F. Abdel-Magid, K. G. Carson, B. D. Harris, C. A. Maryanoff, R. D. Shah, J. Org. Chem., 1996, 61, 3849-3862.
Verwendete Reagenzien: 6-(Boc-amino)hexyl bromide, DMP (Dess-Martin Periodinan).

### Beispiel 18: (CD)-O-N-Stärke

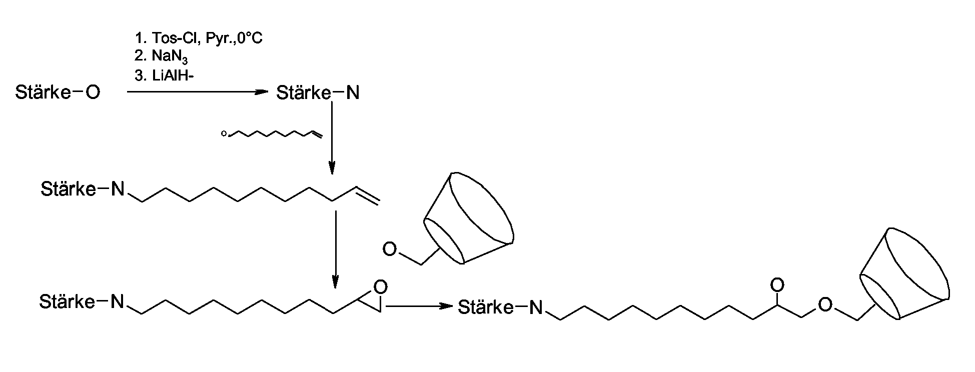

Reduktive Aminierung: March J. Advanced Organic Chemistry pp. 89; JOC, 1996(61) 3849-3862;
Tosylierung von Cellulose: US-A-4286958 S. 11-12 Beispiel 10;
Tosylierung von Stärke: JOC 1963 28 3553.
Verwendete Reagenzien: 10-Undecenal, 3-Chlorperoxybenzoesäure (m-cpba), p-Toluylsulfonylchlorid (Tosylchlorid).

### Beispiel 19: (CD)-O-N-Stärke

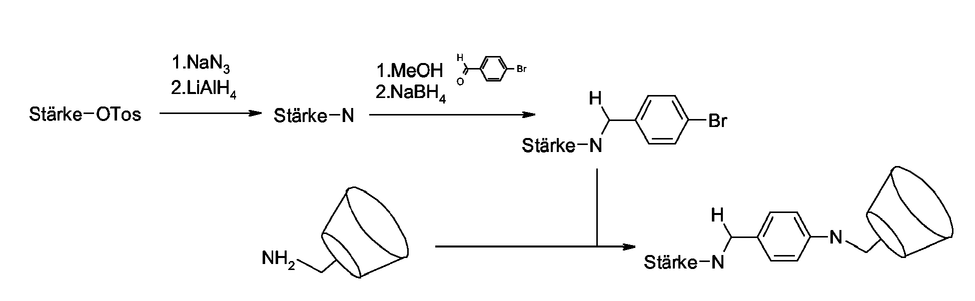

1. Aminoderivate der Stärken. Aminierung von Amylose. M. L. Wolfrom, M. I. Taha, and D.Horton. J. Org. Chem., 28, 3553--3554 (1963);
2. March J. ,Advanced Organic Chemistry pp.898;
3. Reduktive Aminierung: JOC, 1996(61) 3849-3862;
4. Tosylierung von Cellulose: US-A-4286958 S. 11-12 Beispiel 10;
5. Tosylierung von Stärke: JOC 1963 28 3553.
Verwendete Reagenzien: 4-Brombenzaldehyd, Tosychlorid.

### Beispiel 20: (CD)-N-O-Stärke

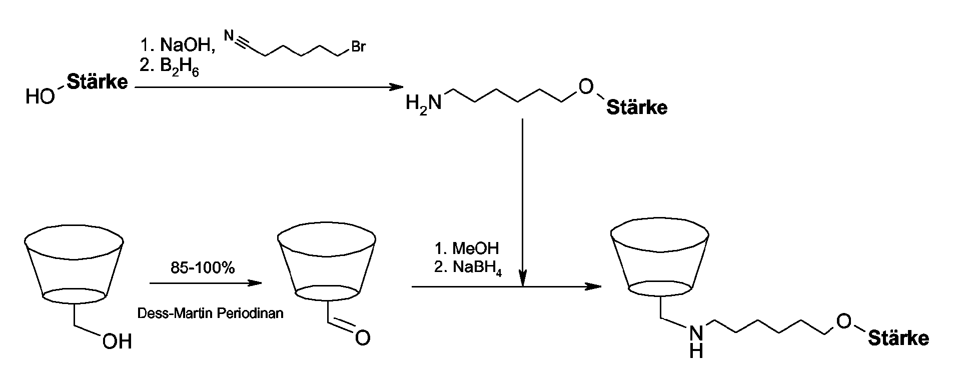

1. Stärke-O-alkyl via Brom: Carbohydrate Research 337 (2002) 2263-2272;
2. Cyclodextrinmonoaldehyd: M. J. Cornwell, J. B. Huff, C. Bieniarz, Tet. Lett. 1995 36(46) 8371-8374;
3. Reduktive Aminierung: A. F. Abdel-Magid, K. G. Carson, B. D. Harris, C. A. Maryanoff, R. D. Shah, J. Org. Chem., 1996, 61, 3849-3862.
Verwendete Reagenzien: 6-Bromhexannitril, Diboran, DMP (Dess-Martin Periodinan).

### Beispiel 21: (CD)-S-O-Stärke

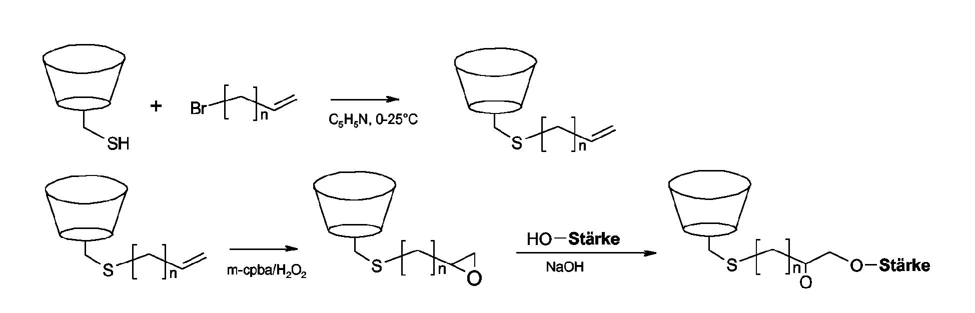

1. Monotosylierung von Cyclodextrinen: L.D. Melton and K.N. Slessor, Carbohydr. Res., 1971 (18) 29-33;
2. Mercaptocyclodextrin: Macromol. Chem. Phys. 1994 195 1719-1732.

### Beispiel 22: (CD)-N-N-Stärke

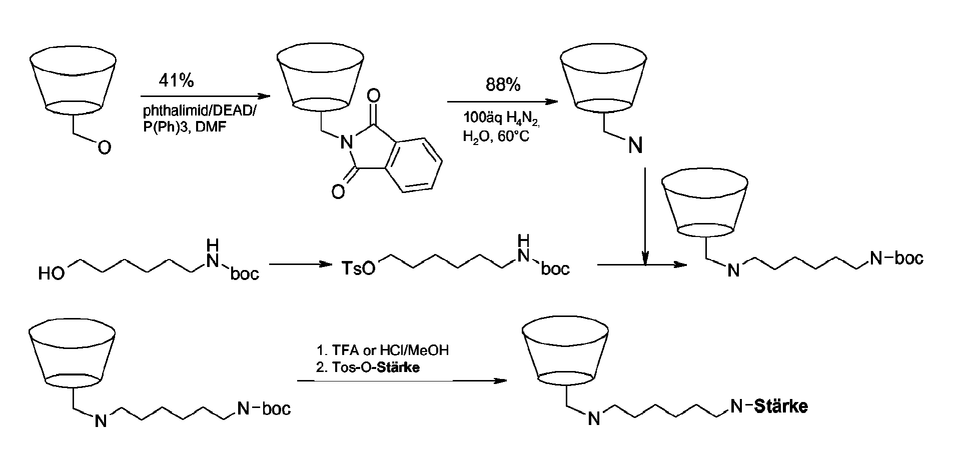

1. Mitsunobu: D.-Q. Yuan, C. Yang, T. Fukuda and K. Fujita Tet. Lett. 2003 44(3) 565-568;
2. Tosylierung von Cellulose: US-A-4286958 S. 11-12 Beispiel 10;
3. Tosylierung von Stärke: JOC 1963 28 3553.

### Beispiel 23: ((CD)-OSi-N-Stärke

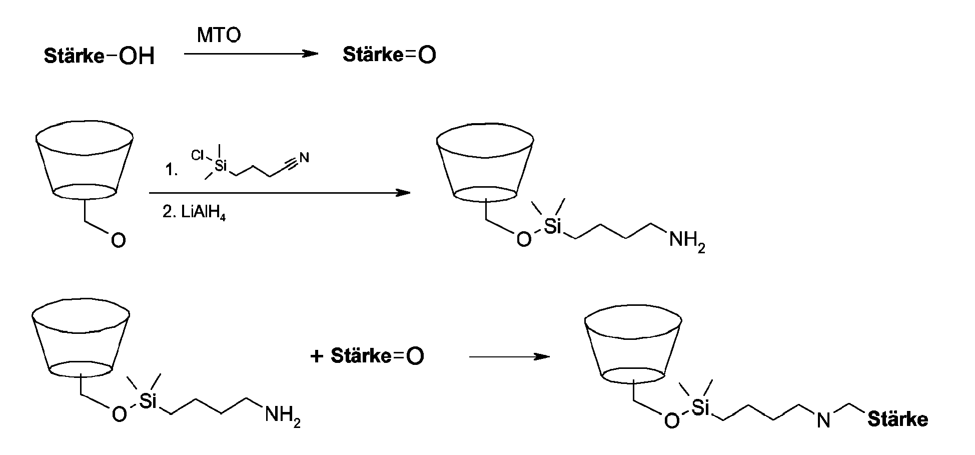

Stärkealdehyde: W. A. Herrmann, J. P. Zollera and R. W. Fischerb J. Organometallic Chem. 579(1-2) 404-407;
Monosilylierung: 'Definiert monofunktionalisierte β-Cyclodextrinderivate' H.Dittmann S 196;
Silylierung primärer Alkohole: Ashton P.R., Königer R., Stoddart J.F., Alker D., Harding V.D. J. Org. Chem. 1996 61 903-908
Reduktive Aminierung: JOC, 1996(61) 3849-3862.
Verwendete Reagenzien: Methyltrioxorhenium(VII), Pyridiniumchlorochromate(PCC), Chlor(3-cyanopropyl)dimethylsilan.

## Patentansprüche

1. Wasserlösliches Addukt umfassend ein Polysaccharid und wenigstens ein Cyclodextrin, die über wenigstens eine Linker-Gruppe kovalent miteinander verknüpft sind.

2. Addukt gemäß Anspruch 1 der allgemeinen Formel (I)
[(CD)-L₁{-R-L₂}ₘ]ₙ-(PS) (I)
wobei
- (CD) ein Cyclodextrin-Rest;
- (PS) ein Polysaccharid-Rest;
- L₁ und L₂ gleich oder unterschiedlich sein können und jeweils eine Linker-Gruppe darstellen;
- R ausgewählt ist aus der Gruppe bestehend aus
- einer Einfachbindung;
- linearen oder verzweigten, gesättigten oder ungesättigten, aliphatischen oder alicyclischen Kohlenwasserstoffgruppen mit 1 bis 22 Kohlenstoffatomen, einer oder mehreren Doppelbindungen, zusätzlichen Heteroatomen, die ausgewählt sind aus der Gruppe bestehend aus O, S, N, Se, Si und P, sowie funktionellen Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatomen;
- aromatischen Gruppen enthaltend 5 bis 12 Kohlenstoffatome;
- gesättigten oder ungesättigten heterocyclischen Gruppen enthaltend 3 bis 10 Kohlenstoffatome und wenigstens ein Heteroatom ausgewählt aus O, S, N, Se, Si und P;
- Aryl-C₁₋₆-alkylen-Gruppen mit 5 bis 12 Kohlenstoffatomen im Arylrest, die substituiert sein können mit wenigstens einer C₁₋₆-Alkylen-Gruppe, wobei sowohl der Arylrest als auch die Alkylen-Gruppe eine oder mehrere Doppelbindungen, zusätzliche Heteroatome, die ausgewählt sind aus der Gruppe bestehend aus O, S, N, Se, Si und P, sowie funktionellen Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatomen aufweisen können;
- m gleich 0 oder 1 und
- n eine ganze Zahl zwischen 1 und 10000 ist.

3. Addukt nach irgendeinem der Ansprüche 1 bis 2, **dadurch gekennzeichnet, dass** die Linker-Gruppen L₁ und L₂ gleich oder ungleich und unabhängig voneinander ausgewählt sein können aus der Gruppe bestehend aus Ester- oder Thioester-, Amino-, Amid-, Aldimin-, Urethan-, Carbonat-, Carbamat-, Thiocarbamat-, Ether-, Thioether-, und Harnstoff-Gruppen.

4. Addukt nach irgendeinem der Ansprüche 1 bis 3, **dadurch gekennzeichnet, dass** sich der Polysaccharid-Rest ableitet aus der Gruppe bestehend aus kationischen, anionischen und neutralen Stärken, Amino(alkyl)stärken, Carboxy(alkyl)stärken, Isocyanato(alkyl)stärken, Hydroxy(alkyl)stärken, Dextranen und Alginaten.

5. Addukt nach irgendeinem der Ansprüche 1 bis 4, **dadurch gekennzeichnet, dass** sich der Cyclodextrin-Rest ableitet aus Verbindungen umfassend wenigstens ein Strukturelement gemäß der allgemeinen Formel II 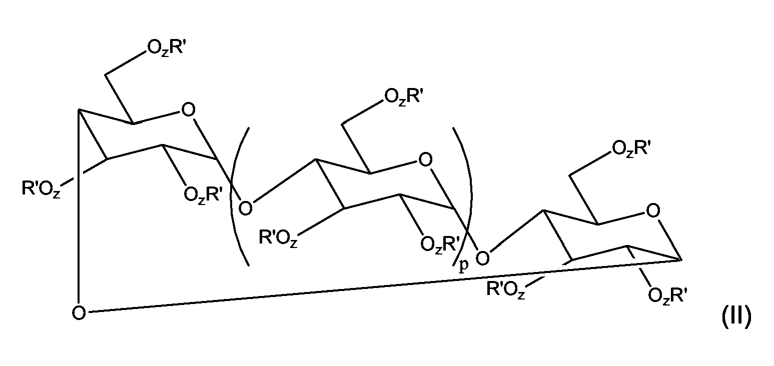 wobei p = 4, 5 oder 6, z = 0 oder 1 ist und die Reste R' unabhängig voneinander ausgewählt sind aus der Gruppe bestehend aus Wasserstoff; Alkyl-, Carboxy(alkyl)- oder Carboxyl-, Sulfo(alkyl)ether-, Hydroxy(alkyl)-, Amino-(alkyl)-, Mercapto(alkyl)- und Isocyanato(alkyl)-Gruppen sowie Halogenatomen.

6. Addukt gemäß irgendeinem der Ansprüche 1 bis 5 **dadurch gekennzeichnet, dass** sich der Cyclodextrin-Rest ableitet aus der Gruppe bestehend aus α-, β- und γ-Cyclodextrinen.

7. Addukt gemäß irgendeinem der Ansprüche 1 bis 6 mit einem Molekulargewicht M_{w}< 500 kDa.

8. Addukt gemäß irgendeiner der nachfolgenden Formeln XIII bis XXIV

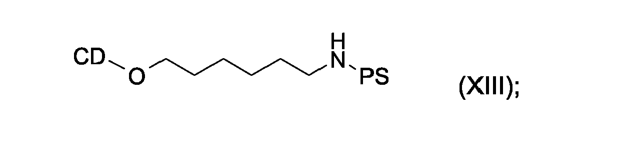

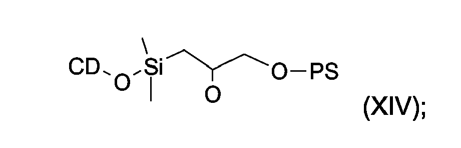

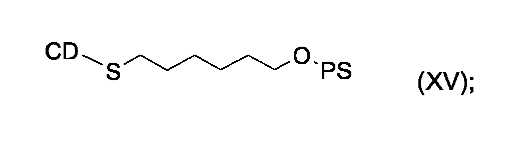

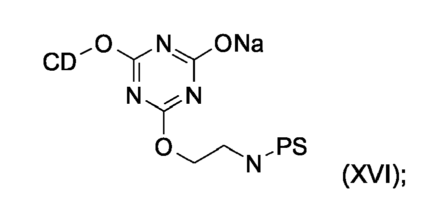

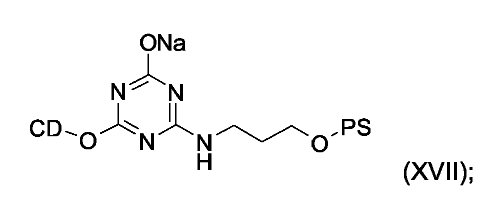

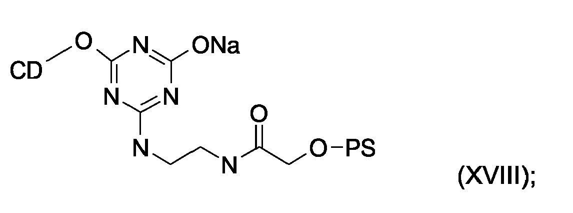

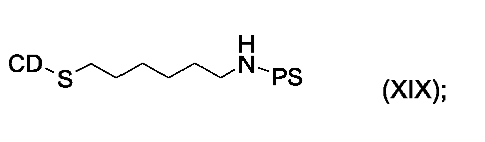

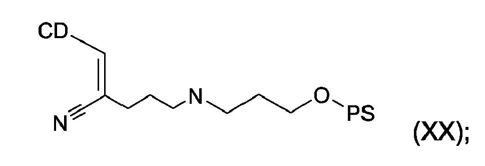

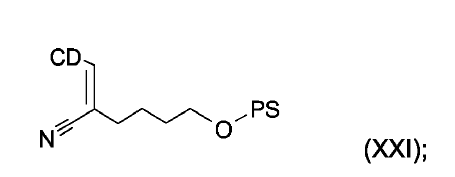

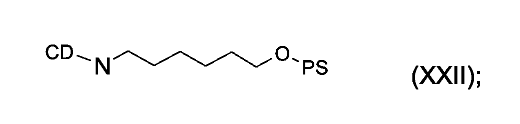

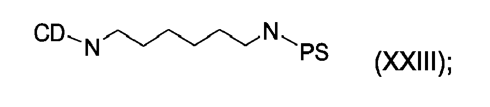

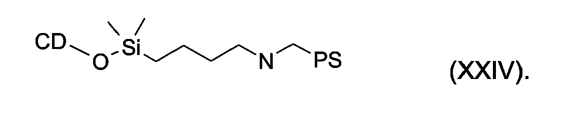

9. Verfahren zur Herstellung des Addukts nach irgendeinem der Ansprüche 1 bis 8, durch kovalente Anknüpfung eines Polysaccharids an ein Cyclodextrin über eine Linker-Gruppe.

10. Verfahren gemäß Anspruch 9 durch Reaktion eines Polysaccharids mit einem Cyclodextrin, wobei sowohl das Polysaccharid als auch das Cyclodextrin wenigstens ein Halogenatom oder eine freie funktionelle Gruppe aufweist, die jeweils ausgewählt ist aus der Gruppe bestehend aus Hydroxy-, Mercapto-, Aldehyd-, Tosylat-, Triflat-, Mesylat-, Carboxyl-, Carbonsäureester-, Carbonsäurehalogenid-, Amino- und Isocyanat-Gruppen; wobei die Auswahl der freien funktionellen Gruppen des Cyclodextrins und des Polysaccharids so zu treffen ist, dass
(i) eine Hydroxy- oder Mercapto-Gruppe mit einer freien funktionellen Gruppe, die ausgewählt ist aus der Gruppe bestehend aus Carboxyl-, Carbonsäureester-, Carbonsäurehalogenid- und Isocyanat-Gruppen unter Bildung einer kovalenten Bindung reagiert; oder dass
ii) eine Amino-Gruppe mit einer freien funktionellen Gruppe, die ausgewählt ist aus der Gruppe bestehend aus Carboxyl-, Aldehyd-, Carbonsäureester-, Carbonsäurehalogenid- und Isocyanat-Gruppen unter Bildung einer kovalenten Bindung reagiert; oder dass
(iii) eine Isocyanat-Gruppen mit einer freien funktionellen Gruppe, die ausgewählt ist aus der Gruppe bestehend aus Carboxyl-, Amino-, Hydroxy- und Mercapto-Gruppen unter Bildung einer kovalenten Bindung reagiert.

11. Verfahren zur Herstellung eines Addukts wie in irgendeinem der Ansprüche 1 bis 8 definiert, durch Reaktion eines Polysaccharids und eines Cyclodextrins mit einem bifunktionellen Linkermolekül.

12. Verfahren nach Anspruch 11, wobei
(i) sowohl das Polysaccharid als auch das Cyclodextrin wenigstens eine freie funktionelle Gruppe aufweisen, die unabhängig voneinander ausgewählt ist aus der Gruppe bestehend aus Carboxyl-, Carbonsäureester-, Carbonsäurehalogenid- und Isocyanat-Gruppen;
und das bifunktionelle Linkermolekül ausgewählt ist aus der Gruppe bestehend aus
- Diaminen der allgemeinen Formel III
R(-NH₂)₂ (III);
- Diolen der allgemeinen Formel IV
R(-OH)₂ (IV);
- Dithiolen der allgemeinen Formel V
R(-SH)₂ (V);
- Aminoalkoholen der allgemeinen Formel VI
H₂N-R-OH (VI);
- Mercaptoalkoholen der allgemeinen Formel VII
HS-R-OH (VII);
oder
(ii) sowohl das Polysaccharid als auch das Cyclodextrin wenigstens eine freie funktionelle Gruppe aufweisen, die unabhängig voneinander ausgewählt sind aus der Gruppe bestehend aus Isocyanat-, Amino- und Hydroxy- oder Mercapto-Gruppen;
und das bifunktionelle Linkermolekül ausgewählt ist aus Dicarbonylverbindungen, die ausgewählt sind aus der Gruppe bestehend aus
- Dicarbonsäuren oder Diestern der allgemeinen Formel VIII
R(-COOR¹)₂ (VIII),
wobei R¹ Wasserstoff oder eine C₁₋₁₀-Alkylgruppe ist;
- Dicarbonsäurehalogeniden der allgemeinen Formel IX
R(-CO-X)₂, (IX),
wobei X = Cl, Br oder I ist;
- Dicarbonsäureanhydriden der allgemeinen Formel X
R(CO)₂O (X);
- Dialdehyden der allgemeinen Formel (XI)
R-(CHO)₂ (XI);
; oder
(iii) sowohl das Polysaccharid als auch das Cyclodextrin wenigstens eine freie funktionelle Gruppe aufweisen, die unabhängig voneinander ausgewählt ist aus der Gruppe bestehend aus Carboxyl-, Hydroxy- und Mercapto-Gruppen;
und das bifunktionelle Linkermolekül ausgewählt ist aus Diisocyanaten der allgemeinen Formel IX
R(-NCO)₂ (XII),
worin die Gruppe R ausgewählt ist aus der Gruppe bestehend aus
- einer Einfachbindung;
- linearen oder verzweigten, gesättigten oder ungesättigten, aliphatischen oder alicyclischen Kohlenwasserstoffgruppen mit 1 bis 22 Kohlenstoffatomen, einer oder mehreren Doppelbindungen, zusätzlichen Heteroatomen, die ausgewählt sind aus der Gruppe bestehend aus O, S, N, Se, Si und P, sowie funktionellen Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatomen;
- aromatischen Gruppen enthaltend 5 bis 12 Kohlenstoffatome;
- gesättigten oder ungesättigten heterocyclischen Gruppen enthaltend 3 bis 10 Kohlenstoffatome und wenigstens ein Heteroatom ausgewählt aus O, S, N, Se, Si und P;
- Aryl-C₁₋₆-alkylen-Gruppen mit 5 bis 12 Kohlenstoffatomen im Arylrest, die substituiert sein können mit wenigstens einer C₁₋₆-Alkylen-Gruppe, wobei sowohl der Arylrest als auch die Alkylen-Gruppe eine oder mehrere Doppelbindungen, zusätzliche Heteroatome, die ausgewählt sind aus der Gruppe bestehend aus O, S, N, Se, Si und P, sowie funktionellen Gruppen, die ausgewählt sind aus der Gruppe bestehend aus Hydroxy-, Alkoholat-, Ether-, Thioether-, Thiol-, Thiolat-, Ester-, Amino-, Amid-, Cyanid- und Carbonyl-Gruppen und/oder Halogenatomen aufweisen können.

13. Verfahren nach irgendeinem der Ansprüche 10 bis 12, **dadurch gekennzeichnet, dass** die freien funktionellen Gruppen des Polysaccharids und des Cyclodextrins gleich sind.

14. Pharmazeutische Zusammensetzung umfassend das Addukt nach irgendeinem der Ansprüche 1 bis 8.

15. Pharmazeutische Zusammensetzung umfassend gemäß Anspruch 14 umfassend ferner wenigstens einen Wirkstoff.

16. Verwendung des Addukts gemäß irgendeinem der Ansprüche 1 bis 8 zur Solubilisierung von Wirkstoffen.

17. Addukt/Wirkstoff Komplex gemäß Anspruch 15, **dadurch gekennzeichnet, dass** der Wirkstoff ausgewählt ist aus der Gruppe bestehend aus Hormonen, Cytostatika, Antibiotika, Fungiziden, Allgemeinanästhetika, Hypnotika, Analgetika und Immunsuppressiva.
